# FASCICULE DE BREVET EUROPEEN

(11) **EP 1 093 796 B1**
(45) Date de publication et mention de la délivrance du brevet: **05.02.2003**
(21) Numéro de dépôt: 00402808.0
(22) Date de dépôt: 11.10.2000
(51) Int. Cl.: A61K 7/42, A61K 7/40, A61K 7/02, A61K 7/06

(54) **Emulsions contenant au moins un filtre uv organique insoluble et un polymere associatif**
Mindestens ein unlösliches organisches UV-Filter und ein assoziatives Polymer enthaltende Emulsionen
Emulsions containing one or more insoluble organic UV-filter and an associative polymer

(30) Priorité: 22.10.1999 FR 9913220
(43) Date de publication de la demande: 25.04.2001
(73) Titulaire: L'OREAL, 75008 Paris (FR)
(72) Inventeur: Candau, Didier, 91570 Bievres (FR); Forestier, Serge, 77410 Claye Souilly (FR); Pisson, Anne-Marie, 91800 Boussy saint Antoine (FR)
(74) Mandataire: Miszputen, Laurent

(56) Documents cités:
- EP-A- 0 987 006
- WO-A-97/03643
- US-A- 5 607 664
- R. LOCHHEAD ET AL.: "Hydrophobically modified "carbopol" resins. A new route to easily prepared, storage-stable, cosmetic lotions that break upon contact with skin." SOAP/COSMETICS/CHEMICAL SPECIALTIES, vol. 63, no. 5, 1987, pages 28-33,84-85, XP000929530
- DATABASE CHEMICAL ABSTRACTS [en ligne] STN; abrégé: 127: 336 467, XP002143622 & JP 09 255523 A (SHISEIDO CO., LTD) 30 septembre 1997 (1997-09-30)
- DATABASE CHEMICAL ABSTRACTS [en ligne] STN; abrégé: 122: 64 010, XP002143606 & JP 06 279252 A (NOEVIR KK) 4 octobre 1994 (1994-10-04)

## Description

La présente invention a pour objet une émulsion cosmétique ou dermatologique, comportant au moins un système photoprotecteur capable de filtrer les rayons UV contenant au moins un filtre UV organique insoluble dans ladite émulsion, sous forme micronisée dont la taille moyenne des particules varie de 0,01 à 2 µm, caractérisée par le fait qu'elle comprend en plus au moins un polymère associatif différent d'un C₈-C₁₆alkylpolyglucoside.

L'invention concerne également ses utilisations pour la fabrication de compositions cosmétiques ou dermatologiques pour la photoprotection de la peau ou des cheveux.

L'invention concerne également ses utilisations pour la fabrication de compositions cosmétiques ou dermatologiques pour la photoprotection de la peau ou des cheveux.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisclaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques, lipophiles et/ou des nanopigments minéraux d'oxydes metalliques, capables d'absorber sélectivement les rayonnements UV nocifs, ces filtres (et leurs quantités) étant sélectionnés en fonction du facteur de protection solaire recherché (le facteur de protection solaire (SPF) s'exprimant mathématiquement par le rapport du temps d'irradiation nécessaire pour atteindre le seuil érythématogène avec le filtre UV au temps nécessaire pour atteindre le seuil érythématogène sans filtre UV). Dans de telles émulsions, les filtres hydrophiles sont présents dans la phase aqueuse et les filtres lipophiles sont présents dans la phase grasse.

Les émulsions huile-dans-eau sont, d'une manière générale, plus appréciées par le consommateur que les émulsions eau-dans-huile, en raison notamment de leur toucher agréable (voisin de l'eau) et de leur présentation sous forme de lait ou de crème non gras ; cependant, elles perdent également plus facilement leur efficacité en protection UV dès lors qu'elles viennent en contact avec l'eau ; en effet, les filtres hydrophiles, ont tendance à disparaître à l'eau, par baignade en mer ou en piscine, sous la douche ou lors de la pratique de sports nautiques ; ainsi, les compositions solaires qui les contiennent, seuls ou associés aux filtres lipophiles, n'apportent plus la protection initiale recherchée dès lors que le substrat (peau ou cheveu) sur lequel elles ont été appliquées vient en contact avec l'eau.

On peut disposer de compositions antisolaires présentant une résistance à l'eau améliorée en mettant en oeuvre des émulsions eau-dans-huile. En effet, un filtre hydrophile est plus rémanent à l'eau au sein d'une émulsion eau-dans-huile qu'au sein d'une émulsion huile-dans-eau. Cependant, comme il a été indiqué plus haut, de telles compositions ne donnent pas encore entièrement satisfaction dans la mesure où elles laissent après application une impression de gras particulièrement désagréable pour l'utilisateur.

Ainsi, le besoin subsiste toujours quant à pouvoir disposer de compositions antisolaires apportant à la peau et/ou aux cheveux une protection solaire efficace, stable dans le temps et résistante à l'eau (rémanence à l'eau) et dont les performances cosmétiques seraient comparables à celles obtenues avec les émulsions huile/eau classiques.

La Demanderesse a découvert de manière surprenante et inattendue que des émulsions particulières contenant au moins un filtre UV organique insoluble sous forme micronisée dans les différentes phases de ces émulsions et au moins un polymère associatif permettaient non seulement d'obtenir des compositions antisolaires dont les performances cosmétiques étaient comparables à celles obtenues généralement avec une composition antisolaire classique sous forme d'émulsion huile/eau mais aussi présentaient une bonne stabilité ainsi qu'une rémanence à l'eau améliorée.

Ces découvertes sont à l'origine de la présente invention.

La présente invention a pour objet une émulsion cosmétique ou dermatologique, comportant au moins un système photoprotecteur capable de filtrer les rayons UV contenant au moins un filtre UV organique insoluble dans ladite émulsion, sous forme micronisée dont la taille moyenne des particules varie de 0,01 à 2 µm, caractérisée par le fait qu'elle comprend en plus au moins un polymère associatif différent d'un C₈-C₁₆alkylpolyglucoside.

Par émulsion cosmétique ou dermatologique, au sens de la présente invention et dans le texte qui suit, on entend toute émulsion dont la phase aqueuse et la phase grasse sont constituées de substances cosmétiquement ou dermatologiquement acceptables pour une application topique sur les matières kératiniques humaines incluant la peau, les cheveux, les cils, les sourcils, les lèvres, les ongles ou les muqueuses.

Par filtre UV organique insoluble, on entend, au sens de la présente invention, des filtres UV organiques insolubles dans les milieux cosmétiques généralement utilisés dans les formulations solaires et plus particulièrement dont la solubilité dans l'eau à 25°C est inférieure à 0,1 % en poids et dont la solubilité dans l'huile de paraffine à 25°C est inférieure à 1% en poids.

Par système photoprotecteur capable de filtrer les rayons UV, on entend par tout système constitué d'un ou plusieurs composés organiques et/ou composés minéraux filtrant les radiations UVA et/ou UV-B.

Par polymère associatif, au sens de la présente invention et dans le texte qui suit, on entend tout polymère amphiphile comportant dans sa structure au moins une chaîne grasse et au moins une portion hydrophile.

La présente invention a également pour objet l'utilisation de l'émulsion pour la fabrication de compositions cosmétiques pour la protection de la peau et/ou des cheveux contre le rayonnement ultraviolet, en particulier le rayonnement solaire.

D'autres caractéristiques, aspects et avantages de la présente invention apparaîtront à la lecture de la description détaillée qui va suivre.

Les filtres organiques insolubles selon l'invention se présentent sous forme micronisée. La taille moyenne des particules varie de 0,01 à 2µm et plus préférentiellement de 0,02 à 1,5 µm et plus particulièrement de 0,05 à 1,0 µm.

Les filtres organiques insolubles selon l'invention peuvent être amenés sous la forme particulaire souhaitée par tout moyen ad-hoc tel que notamment broyage à sec ou en milieu solvant, tamisage, atomisation, micronisation, pulvérisation.

Les filtres organiques insolubles selon l'invention sous forme micronisée. peuvent en particulier être obtenus par un procédé de broyage d'un filtre UV insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893119 faisant partie intégrante de la description. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure CₙH₂ₙ₊₁ O(C₆H₁₀O₅)ₓH dans laquelle n est un entier de 8 à 16 et x est le degré moyen de polymérisation de l'unité (C₆H₁₀O₅) et varie de 1,4 à 1,6. Ils peuvent être choisis parmi des esters en C₁-C₁₂ d'un composé de structure CₙH₂ₙ₊₁ O(C₆H₁₀O₅)ₓH et plus précisément un ester obtenu par réaction d'un acide carboxylique en C₁-C₁₂ tel que l'acide formique, acétique, propionique, butyrique, sulfosuccinique, citrique ou tartrique avec une ou plusieurs fonctions OH libres sur l'unité glucoside (C₆H₁₀O₅). Lesdits tensio-actifs sont utilisés en général à une concentration de allant de 1 à 50% en poids et plus préférentiellement de 5 à 40% en poids par rapport au filtre insoluble dans sa forme micronisée.

Les polymères associatifs conformes à la présente invention peuvent être anioniques, non-ioniques ou cationiques ou amphotères.

Parmi les polymères anioniques associatifs, on peut citer ceux comportant au moins un motif hydrophile, et au moins un motif éther d'allyle à chaîne grasse, plus particulièrement parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyle à chaîne grasse correspond au monomère de formule (I) suivante :

CH₂ = C(R')CH₂ O Bₙ R (I)

dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone. Un motif de formule (I) plus particulièrement préféré selon la présente invention est un motif dans lequel R' désigne H, n est égal à 10, et R désigne un radical stéaryl (C₁₈).

Des polymères amphiphiles anioniques de ce type sont décrits et préparés, selon un procédé de polymérisation en émulsion, dans le brevet EP-0 216 479.

Parmi ces polymères anioniques associatifs, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

Parmi les polymères anioniques associatifs, on peut citer également les polymères anioniques comportant au moins un motif hydrophile de type acide carboxylique insaturé oléfinique, et au moins un motif hydrophobe exclusivement de type ester d'alkyl (C₁₀-C₃₀) d'acide carboxylique insaturé.

De préférence, ces polymères sont choisis parmi ceux dont le motif hydrophile de type acide carboxylique insaturé oléfinique correspond au monomère de formule (II) suivante : 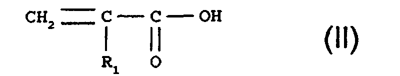 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante : 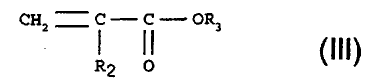 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle et les méthacrylates correspondants, le méthacrylate de lauryle, le méthacrylate de stéaryle, le méthacrylate de décyle, le méthacrylate d'isodécyle.

Des polymères anioniques de ce type sont par exemple décrits et préparés, selon les brevets US-3 915 921 et 4 509 949.

Parmi ce type de polymères anioniques associatifs, on utilisera plus particulièrement des polymères formés à partir d'un mélange de monomères comprenant :
(i) essentiellement de l'acide acrylique,
(ii) un ester de formule (III) suivante : 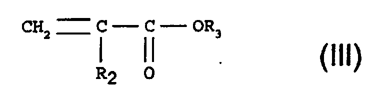 dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères anioniques associatifs, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.

Parmi les polymères anioniques associatifs, on peut citer également les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608 par la société NEWPHASE TECHNOLOGIES.

Parmi les polymères anioniques associatifs, on peut citer également les terpolymères acryliques comprenant :
(a) environ 20% à 70% en poids d'un acide carboxylique à insaturation α,β-monoéthylénique
(b) environ 20 à 80% en poids d'un monomère à insaturation α,β-monoéthylénique non-tensio-actif différent de (a)
(c) environ 0,5 à 60% en poids d'un mono-uréthane non-ionique qui est le produit de réaction d'un tensio-actif monohydrique avec un monoisocyanate à insaturation monoéthylénique tels que ceux décrits dans la demande de brevet EP-A-0173109 et plus particulièrement celui décrit dans l'exemple 3 à savoir un terpolymère acide méthacrylique/acrylate de méthyle/diméthyl métaisopropényl benzyl icocyanate d'alcool béhényle éthoxylé (40OE) en dispersion aqueuse à 25%.

Les polymères associatifs non-ioniques, utilisés selon l'invention, sont choisis de préférence parmi :
(1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
(2) les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
(3) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
(4) les copolymères de vinyl pyrrolidone et de monomères hydrophobes à chaîne grasse ;
   on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
(5) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
(6) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

De préférence, les polyéthers polyuréthanes comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

De préférence, les polyéthers polyuréthanes non-ioniques associatifs sont des copolymères triblocs dont la séquence hydrophile est une chaîne polyoxyéthylénée comportant de 50 à 1000 groupements oxyéthylénés. En polyéthers polyuréthanes non-ioniques comportent une liaison uréthanne entre les séquences hydrophiles, d'où l'origine du nom.

Par extension figurent aussi parmi les polyéthers polyuréthanes non-ioniques associatifs, ceux dont les séquence hydrophiles sont liées par d'autres liaisons chimiques aux séquences lipophiles.

A titre d'exemples de polyéthers polyuréthanes non-ioniques associatifs utilisables dans l'invention, on peut citer le polymère C₁₆-OE₁₂₀-C₁₆ vendu par la société SERVO DELDEN (sous le nom SER-AD FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 , 204 ou 212; ainsi que l'Acrysol RM 184, l'Aculyn 44 et l'Aculyn 46 de la société RHOM & HAAS.

On peut également citer le produit ELFACOS T210 à chaîne alkyle en C₁₂₋₁₄ et le produit ELFACOS T212 à chaîne alkyle en C₁₈ de chez AKZO.

Le produit DW 1206B de chez RHOM & HAAS à chaîne alkyle en C₂₀ et à liaison uréthanne, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le SER-AD FX1010 et le SER-AD 1035 vendus par la société HULS, le Rhéolate 255, le Rhéolate 278 et le Rhéolate 244 vendus par la société RHEOX. On peut aussi utiliser le produit DW 1206F et le DW 1206J.

Les polyuréthanes utilisables dans l'invention sont en particulier ceux décrits dans l'article de G. Fonnum, J. Bakke et Fk. Hansen - Colloid Polym. Sci 271, 380.389 (1993).

Les polymères associatifs cationiques utilisés dans la présente invention sont choisis de préférence parmi les dérivés de cellulose quaternisée et les polyacrylates à groupements latéraux aminés.

Les dérivés de cellulose quaternisée sont, en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les polyacrylates à groupements latéraux aminés, quaternisés ou non, possèdent par exemple des groupements hydrophobes du type stéareth 20 (alcool stéarylique polyoxyéthyléné(20)).

Les radicaux alkyle portés par les celluloses ou hydroxyéthylcelluloses quatemisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quatemisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

Comme exemples de polyacrylates à chaînes latérales aminées, on peut citer les polymères 8781-121B ou 9492-103 de la société NATIONAL STARCH.

Parmi les polymères amphotères associatifs de l'invention, on peut citer les polymères amphotères , réticulés ou non réticulés, branchés ou non branchés, susceptibles d'être obtenus par la copolymérisation
1) d'au moins un monomère de formule (IVa) ou (IVb): 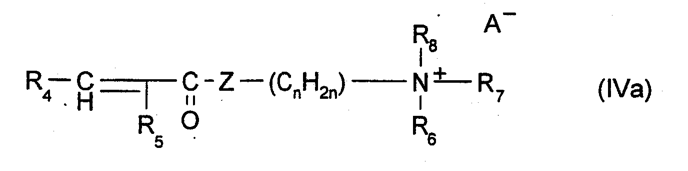 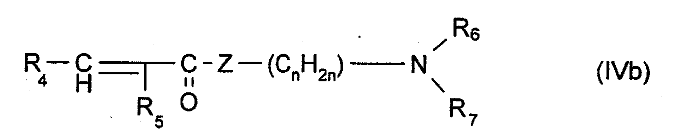 dans lesquelles R₄ et R₅, identiques ou différents, représentent un atome d'hydrogène ou un radical méthyle,
   R₆, R₇ et R₈, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure.
2) d'au moins un monomère de formule (V) : 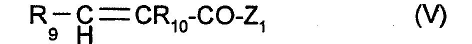 dans laquelle R₉ et R₁₀, identiques ou différents, représentent un atome d'hydrogène ou un radical méthyle;
   Z₁ représente un groupe OH ou un groupe NHC(CH₃)₂CH₂SO₃H;
3) d'au moins un monomère de formule (VI) 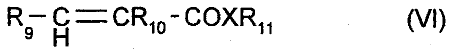 dans laquelle R₉ et R₁₀, identiques ou différents, représentent un atome d'hydrogène ou un radical méthyle, X désigne un atome d'oxygène ou d'azote et R₁₁ désigne un radical alkyle linéaire ou ramifié ayant de 1 à 30 atomes de carbone;
4) éventuellement au moins un agent de réticulation ou de branchement ; l'un au moins des monomères de formule (IVa), (IVb) ou (VI) comportant au moins une chaîne grasse ayant de 8 à 30 atomes de carbone et lesdits composés des monomères de formule (IVa), (IVb), (V) et (VI) pouvant être quaternisés par exemple par un halogénure d'alkyle en C₁-C₄ ou un sulfate de dialkyle en C₁-C₄.

Les monomères de formule (IVa) et (IVb) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,, éventuellement quaternisés par exemple par un halogénure d'alkyle en C1-C4 ou un sulfate de dialkyle en C₁-C₄.

Plus particulièrement, le monomère de formule (IVa) est choisi parmi le chlorure d'acrylamidopropyl triméthyl ammonium et le chlorure de méthacrylamidopropyl triméthyl ammonium.

Les composés de formule (V) de la présente invention sont choisis, de préférence, dans le groupe constitué par l'acide acrylique, l'acide méthacrylique, l'acide crotonique, l'acide méthyl-2 crotonique, l'acide 2-acrylamido-2-méthylpropane sulfonique et l'acide 2-méthacrylamido-2-méthylpropane sulfonique. Plus particulièrement, le monomère de formule (V) est l'acide acrylique.

Les monomères de formule (VI) de la présente invention sont choisis, de préférence, dans le groupe constitué des acrylates ou méthacrylate d'alkyle en C12-C22 et plus particulièrement en C₁₆-C₁₈.

L'agent de réticulation ou de branchement est de préférence choisi parmi le N,N'-méthylène bis-acrylamide, le chlorure de triallyl méthyl ammonium, le méthacrylate d'allyle, le n-méthylolacrylamide, les diméthacrylate de polyéthylène glycols, le diméthacrylate d'éthylène glycol, le diméthacrylate de diéthylène glycol, le diméthacrylate de 1,6-hexanediol et l'allyl sucrose.

Les polymères selon l'invention peuvent également contenir d'autre monomères tels que des monomères non ioniques et en particulier tels que les acrylates ou méthacrylates d'alkyle en C₁-C₄.

Le rapport du nombre de charges cationiques/charges anioniques dans ces polymères amphotères est de préférence égal à environ 1.

Les poids moléculaires moyen en poids des polymères amphotères associatifs, présentent une masse moléculaire moyenne en poids supérieure à 500, de préférence comprise entre 10000 et 10000000 et encore plus préférentiellement entre 100000 et 8000000.

De préférence les polymères amphotères associatifs de l'invention contiennent de 1 à 99 moles %, plus préférentiellement de 20 à 95 moles% et encore plus préférentiellement de 25 à 75 moles % de composé(s) de formule (IVa) ou (IVb). Ils contiennent aussi de préférence de 1 à 80 moles %, plus préférentiellement de 5 à 80 moles% et encore plus préférentiellement de 25 à 75 moles% de composé(s) de formule (V). La teneur en composé (s) de formule (VI) est de préférence comprise entre 0,1 et 70 moles%, plus préférentiellement entre 1 à 50 moles% et encore plus préférentiellement entre 1 à 10 moles%. L'agent de réticulation ou de branchement lorsqu'il est présent est de préférence compris entre 0,0001 et 1 mole% et plus préférentiellement encore entre 0,0001 et 0,1 mole%.

De préférence le rapport molaire entre le ou les composés de formules (IVa) ou (IVb) et le ou les composés de formule (V) varie de 20 :80 à 95 :5 et plus préférentiellement de 25 :75 à 75 :25.

Les polymères amphotères associatifs selon l'invention sont par exemple décrits dans la demande de brevet WO9844012.

Les polymères amphotères particulièrement préférés selon l'invention sont choisis parmi les copolymères acide acrylique/chlorure d'acrylamidopropyl triméthyl ammonium/méthacrylate de stéaryle.

Selon une forme particulière de l'invention, le ou les polymères associatifs jouent le rôle d'émulsionnant de la phase huileuse dans la phase aqueuse. Lorsque le polymère associatif est utilisé comme seul émulsionnant, il est très avantageusement présent en quantité allant de 0,1 à 20 % du poids total de la composition, de préférence en une quantité allant de 0,5 à 10 %.

Les filtres UV organiques insolubles conformes à l'invention peuvent être choisis notamment parmi les filtres UV organiques du type oxanilide, du type triazine, du type triazole, du type amide vinylique, du type cinnamide.

Parmi les filtres UV du type oxanilide conformes à l'invention, on peut citer ceux répondant à la structure : 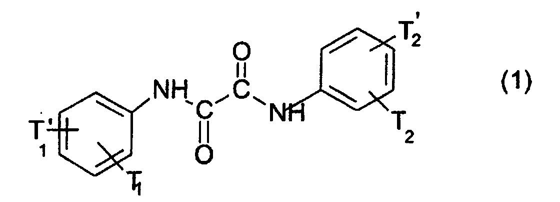 dans laquelle T₁, T'₁, T₂ et T'₂ désignent, identiques et différents, un radical alkyle en C₁-C₈ ou un radical alcoxy en C₁-C₈. Ces composés sont décrits dans la demande de brevet WO95/22959.

A titre d'exemples , on peut citer les produits commerciaux TINUVIN 315 et TINUVIN 312 vendus par la Société CIBA-GEIGY et respectivement de structure :

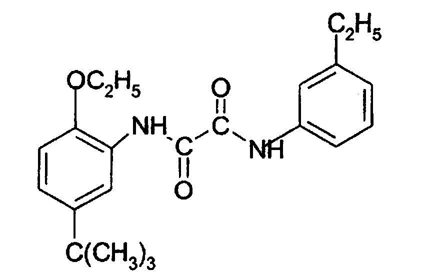

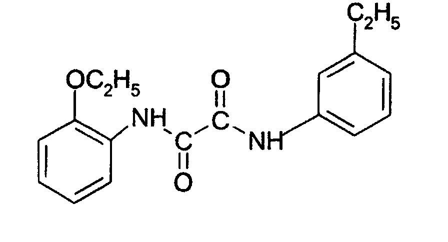

Les dérivés de 1,3,5-triazine conformes à l'invention préférentiels répondent à la formule générale suivante : 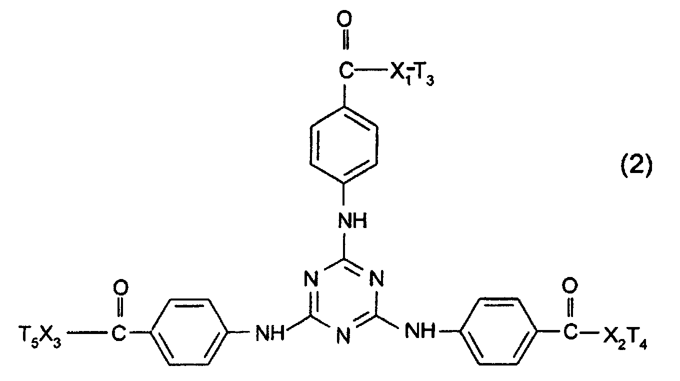 dans laquelle :
- X₁, X₂ et X₃, identiques ou différents, représentent l'oxygène ou un radical -NZ-;
- les radicaux Z, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- T₃, T₄ et T₅, identiques ou différents, sont choisis parmi : l'hydrogène; un métal alcalin; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical polyoxyéthyléné comprenant de 1 à 6 unités d'oxyde d'éthylène et dont le groupe OH terminal est méthylé ; un radical de formule (3), (4) ou (5) suivantes : 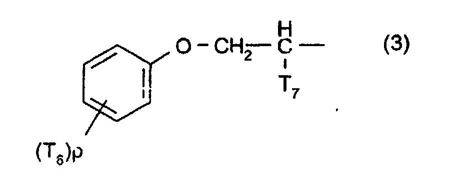 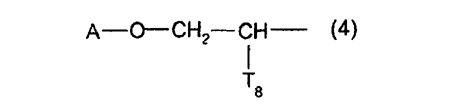 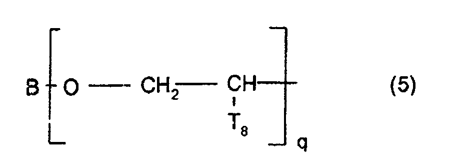 dans lesquelles :
   - T₆ est l'hydrogène ou un radical méthyle ;
   - T₇ est un radical alkyle en C₁-C₉ ;
   - p est un nombre entier allant de 0 à 3 ;
   - q est un nombre entier allant de 1 à 10 ;
   - A est un radical alkyle en C₄-C₈ ou un radical cycloalkyle en C₅-C₈ ;
   - B est choisi parmi : un radical alkyle linéaire ou ramifié en C₁-C₈ ; un radical cycloalkyle en C₅-C₈ ; un radical aryle éventuellement substitué par un ou plusieurs radicaux alkyle en C₁-C₄ ;
   - T₈ est l'hydrogène ou un radical méthyle ;

Une première famille préférée de dérivés de 1,3,5-triazine est celle, notamment décrite dans le document EP-A-0517104 (dont les enseignements sont, pour ce qui touche à la définition même de ces produits, totalement inclus à titre de référence dans la présente description) des 1,3,5-triazines répondant à la formule (2) ci-dessus et présentant l'ensemble des caractéristiques suivantes :
- X₁, X₂ et X₃ sont identiques et représentent l'oxygène ;
- T₃ est choisi parmi : un radical cycloalkyle en C₅-C₁₂ éventuellement substitué par un ou plusieurs radicaux alkyles en C₁-C₄ ; un radical de formule (2), (3) ou (4) ci-dessus dans lesquelles :

- B est un radical alkyle en C₁-C₄ ;
- T₈ est le radical méthyle ;
- T₄ et T₅, identiques ou différents, sont choisis parmi : l'hydrogène ; un métal alcalin ; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical de formule (3), (4) ou (5) ci-dessus dans lesquelles :

- B est un radical alkyle en C₁-C₄ ;
- T₈ est le radical méthyle.

Une deuxième famille préférée de dérivés de 1,3,5-triazine selon l'invention est celle, notamment décrite dans le document EP-A-0570838 (dont les enseignements sont, pour ce qui touche à la définition même de ces produits, totalement inclus à titre de référence dans la présente description) des 1,3,5-triazines répondant à la formule (2) et présentant l'ensemble des caractéristiques suivantes :
- X₁ est l'oxygène ; X₂ est le radical -NH- ou l'oxygène ;
- X₃ est le radical -NH- ;
- T₅ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- T₃ est choisi parmi : l'hydrogène; un métal alcalin ; un radical ammonium; un radical de formule (5) ; un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- si X₂ est le radical -NH-, alors T₄ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄ ;
- si X₂ est l'oxygène, alors T₄ est choisi parmi l'hydrogène ; un métal alcalin ; un radical ammonium; un radical de formule (5) ; un radical alkyle linéaire ou ramifié en C₁-C₁₈ ; un radical cycloalkyle en C₅-C₁₂ éventuellement substitué avec un ou plusieurs radicaux alkyles en C₁-C₄.

Une troisième famille préférée de dérivés de 1,3,5-triazine selon l'invention est celle, notamment décrite dans le document EP-A-0796851 dont les enseignements sont, pour ce qui touche à la définition même de ces produits, totalement inclus à titre de référence dans la présente description) des 1,3,5-triazines répondant à la formule (2) et présentant l'ensemble des caractéristiques suivantes :
- X₁, X₂ et X₃ désignent simultanément -NZ- ;
- les radicaux Z, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- T₃, T₄ et T₅, identiques ou différents, désignent l'hydrogène ou un radical Z.

Ces filtres UV organiques du type triazine sont décrits dans les brevets US4617390, EP517104, EP570838, EP796851 (faisant partie intégrante du contenu de la description).

Parmi ces filtres UV du type triazine de formule (2), on citera plus particulièrement :
- la 2,4,6-tris[p-(2'-éthylhexyl-1'-oxycarbonyl)-anilino]-1,3,5-triazine qui est un filtre connu en soi, actif dans l'UV-B, se présentant sous une forme solide, et qui est vendu notamment sous la dénomination commerciale de "UVINUL T150" par la Société BASF. Ce produit répond à la formule suivante : 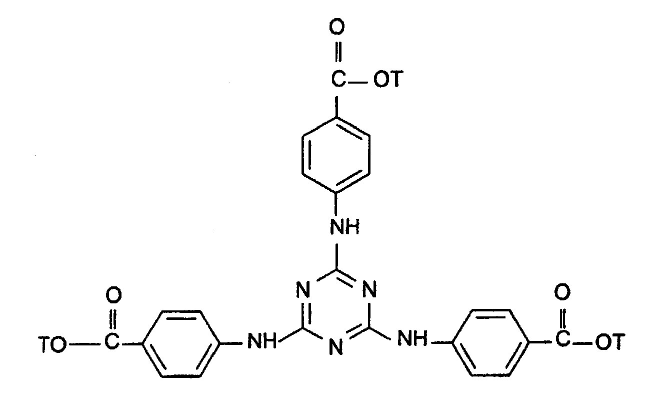 dans laquelle T désigne un radical 2-éthylhexyle ;
- la 2-[(p-(tertiobutylamido)anilino]-4,6-bis-[(p-(2'-éthylhexyl-1'-oxycarbonyl)anilino]- 1,3,5-triazine, de structure suivante : 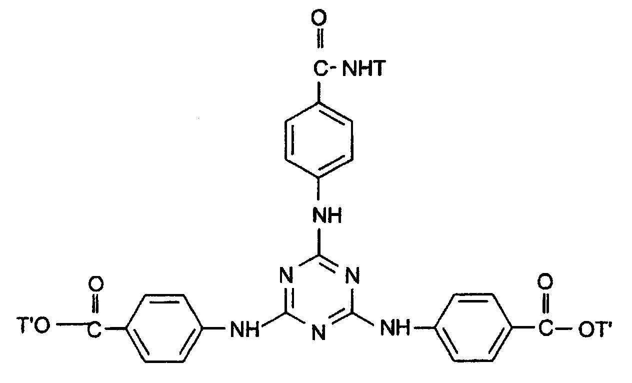 dans laquelle T' désigne un radical 2-éthylhexyle et T désigne un radical tert-butyle.

Parmi les filtres UV insolubles du type triazine conformes à l'invention, on peut également mentionner les dérivés insolubles de s-triazine portant des groupements benzalmalonates et/ou phenylcyanoacrylates tels que ceux décrits dans la demande EP-A-0790243 (faisant partie intégrante du contenu de la description).

Parmi ces filtres UV du type triazine, on citera plus particulièrement les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

Parmi les filtres UV insolubles du type triazine conformes à l'invention, on peut également mentionner ceux répondant à la formule (6) suivante : 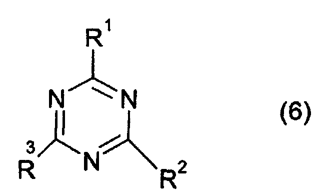 dans laquelle R¹, R², R³, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyalkyle, C₅-C₈cycloalkyle, un groupe méthylidènecamphre, un groupe -(CH=CH)ₙ(CO)-OR⁴, avec R⁴ soit C₁-C₁₈alkyle soit cinnamyle, et n vaut 0 ou 1.

Ces composés sont décrits dans WO 97/03642, GB 2286774, EP-743309, WO 98/22447, GB 2319523 (faisant partie intégrante du contenu de la description).

Parmi les filtres UV insolubles du type triazine conformes à l'invention, on peut encore mentionner les dérivés insolubles de s-triazine portant des groupements benzotriazoles et/ou benzothiazoles tels que ceux décrits dans la demande WO98/25922 (faisant partie intégrante du contenu de la description).

Parmi ces composés, on peut citer plus particulièrement :
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

Parmi les filtres UV organiques du type triazole conformes à l'invention, on peut citer ceux de formule suivante (7) tels que décrits dans la demande WO95/22959 (faisant partie intégrante du contenu de la description) : 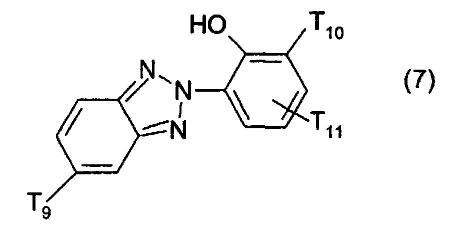 dans laquelle T₉ désigne un atome d'hydrogène ou un radical alkyle en C₁-C₁₈ ; T₁₀ et T₁₁, identiques ou différents, désignent un radical alkyle en C₁-C₁₈ éventuellement substitué par un phényle.

A titre d'exemple de composés de formule (7), on peut citer les produits commerciaux TINUVIN 328, 320, 234 et 350 de la Société CIBA-GEIGY de structure suivante :

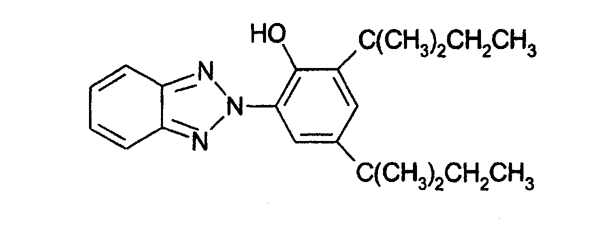

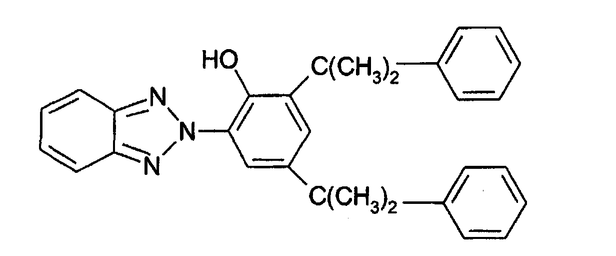

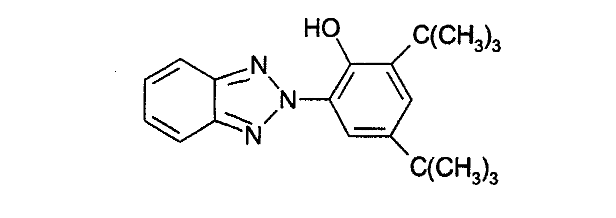

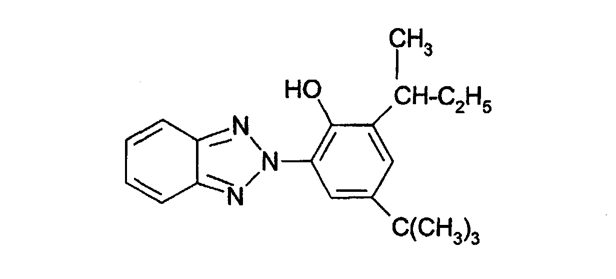

Parmi les filtres UV organiques du type triazole conformes à l'invention, on peut citer les composés tels que décrits dans les brevets. US 5 687 521, US 5 687 521, US 5 373 037, US 5 362 881 et en particulier le [2,4'-dihydroxy-3-(2H-benzotriazol-2-yl)-5-(1,1,3,3-tétraméthylbutyl)-2'-n-octoxy-5'-benzoyl] diphénylméthane vendu sous le nom MIXXIM PB30 par la société FAIRMOUNT CHEMICAL de structure :

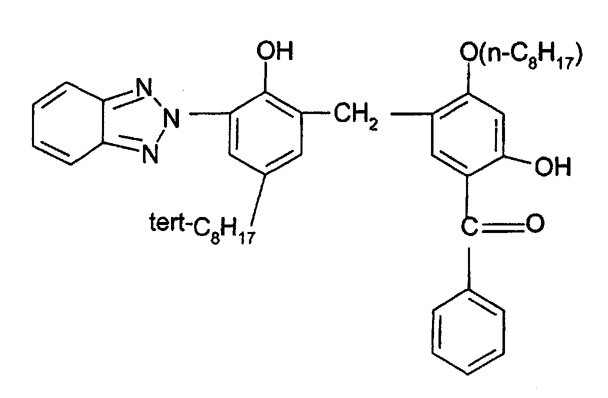

Parmi les filtres UV organiques du type benzotriazole conformes à l'invention, on peut citer les dérivés de méthylène bis-(hydroxyphényl benzotriazole) de structure suivante : 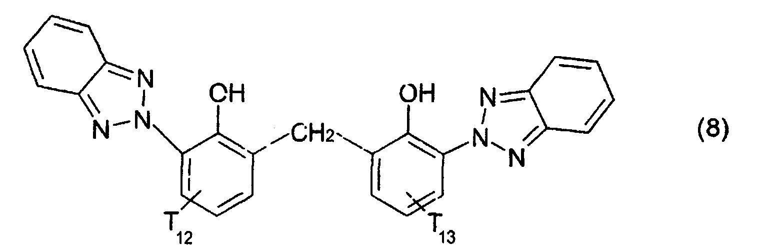 dans laquelle les radicaux T₁₂ et T₁₃, identiques ou différents, désignent un radical alkyle en C₁-C₁₈ pouvant être substitué par un ou plusieurs radicaux choisis parmi alkyle en C₁-C₄, cycloalkyle en C₅-C₁₂ ou un reste aryle. Ces composés sont connus en soi et décrits dans les demandes US 5237 071, US 5 166 355, GB-A-2 303 549, DE 197 26 184 et EP-A-893 119 (faisant partie intégrante de la description).

Dans la formule (8) définie ci-dessus : les groupes alkyle en C₁-C₁₈ peuvent être linéaires ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexydécyle, ou octadécyle ; les groupes cycloalkyle en C₅-C₁₂ sont par exemple cyclopentyle, cyclohexyle, cyclooctyle ; les groupes aryle sont par exemple phényle, benzyle.

Parmi les composés de formule (8), on préfère plus particulièrement ceux de structure suivante :

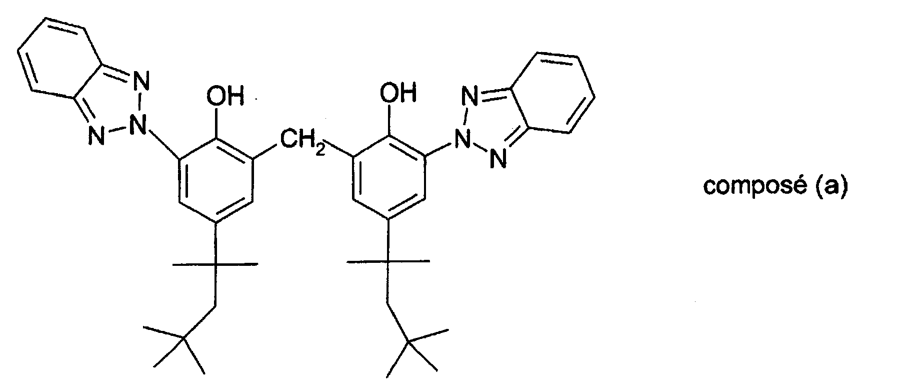

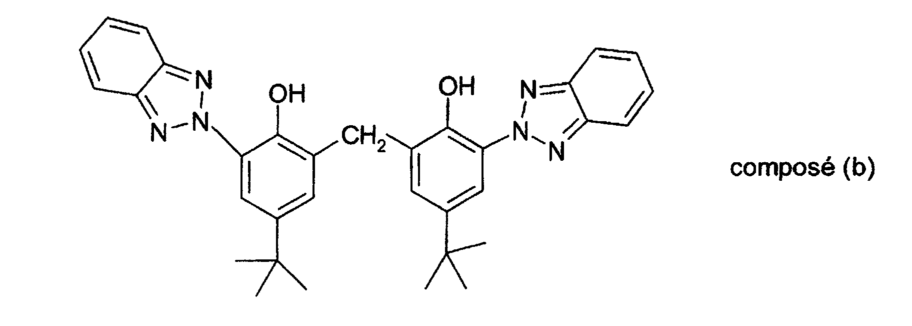

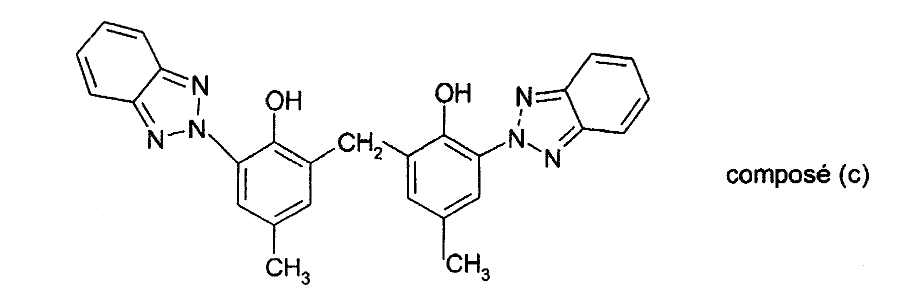

Le composé (a) de nomenclature 2,2'-méthylène-bis-[6-(2H-benzotriazol-2-yl)-4-(1,1,3,3-tetraméthylbutyl)phénol] est vendu sous le nom MIXXIM BB/100 par le société FAIRMOUNT CHEMICAL. Il est vendu sous forme micronisée sous le nom de TINOSORB M par la Société CIBA GEIGY.

Le composé (c) de nomenclature 2,2'-méthylène-bis-[6-(2H-benzotriazol-2-yl)-4-(methyl)phénol] est vendu sous le nom MIXXIM BB/200 par le société FAIRMOUNT CHEMICAL .

Parmi les filtres organiques du type amide vinylique conformes à l'invention, on peut citer par exemple les composés de formules suivante qui sont décrits dans la demande WO95/22959 (faisant partie intégrante du contenu de la description) :

T₁₄-(Y)r-C(=O)-C(T₁₅)=C(T₁₆)-N(T₁₇)(T₁₈) (9)

dans laquelle T₁₄ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₉ où T₁₉ est un alkyle en C₁-C₁₈ ; T₁₅, T₁₆, T₁₇ et T₁₈ identiques ou différents désignent un radical alkyle en C₁-C₁₈, de préférence en C₁-C₅ ou un atome d'hydrogène ; Y est N ou O et r vaut 0 ou 1.

Parmi ces composés, on citera plus particulièrement :
- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

Parmi les filtres organiques insolubles du type cinnamamide, on peut citer également les composés tels que décrits dans la demande WO95/22959 (faisant partie intégrante du contenu de la description) et répondant à la structure suivante : 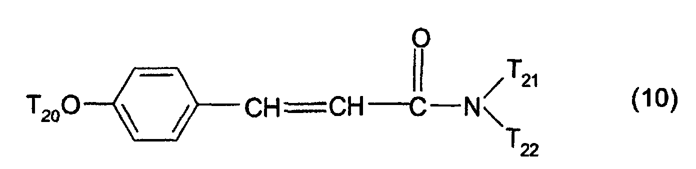 dans laquelle OT₂₀ est un radical hydroxy ou alcoxy en C₁-C₄, de préférence méthoxy ou éthoxy ; T₂₁ est hydrogène, alkyle en C₁-C₄, de préférence méthyle ou éthyle ; T₂₂ est un groupe -(CONH)s-phényle ou s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₂₃ où T₂₃ est un alkyle en C₁-C₁₈ et plus préférentiellement T₂₃ est un groupe phényle, 4-méthoxyphényle ou phénylaminocarbonyle.

On peut également citer les dimères cinnamamides tels que ceux décrits dans le brevet US 5888481 comme par exemple le composé de structure :

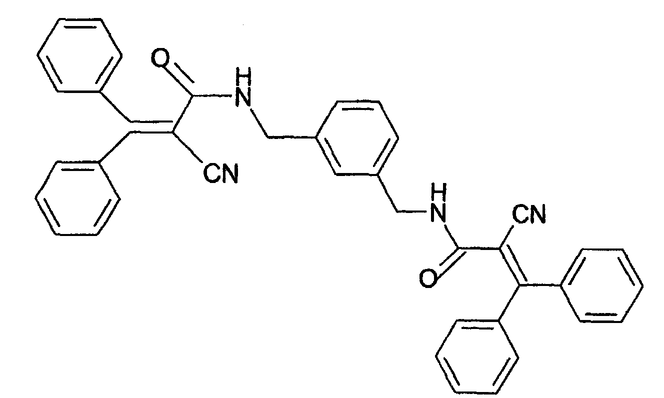

Une autre famille particulière de filtres organiques insolubles conformes à l'invention sont les sels de métaux polyvalents (par exemple Ca ²⁺, Zn²⁺, Mg²⁺, Ba ²⁺, Al³⁺ ou Zr⁴⁺) de filtres organiques sulfoniques ou carboxyliques tels que les sels de métaux polyvalents de dérivés sulfonés de benzylidène camphre tels que ceux décrits dans la demande FR-A 2 639 347 ; les sels de métaux polyvalents de dérivés sulfonés de benzimidazole tels que ceux décrits dans la demande EP-A-893 119 ; les sels de métaux polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que décrits dans les demandes de brevet WO93/10753, WO93/11095 et WO95/05150.

Le ou les filtres organiques insolubles micronisés selon l'invention sont généralement présents dans les compositions filtrantes selon l'invention à une concentration totale comprise entre 0,1 et 15 % en poids environ, et de préférence entre 0,2 et 10 % en poids environ, par rapport au poids total de la composition.

Un autre objet de l'invention consiste en des compositions cosmétiques ou dermatologiques caractérisées par le fait qu'elle comprennent au moins une émulsion telle que définie précédemment.

Les compositions cosmétiques antisolaires selon l'invention peuvent bien entendu contenir un ou plusieurs filtres organiques complémentaires actifs dans l'UVA et/ou l'UVB (absorbeurs), solubles dans au moins l'une des phases des compositions. Ces filtres complémentaires peuvent être notamment choisis parmi les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP 933376 ; les dérivés de la benzophénone ; les dimères dérivés d'α-alkylstyrène tels que ceux décrits dans la demande DE 198 55 649 ; les dérivés de β,β'-diphénylacrylate, les dérivés de benzimidazole ; les dérivés bis-benzoazolyle tels que décrits dans les brevets EP-A-0669323 et US 2,463,264; les dérivés de l'acide p-aminobenzoïque ; les polymères filtres et silicones filtres tels que ceux décrits notamment dans la demande WO-93/04665.

Comme exemples de filtres solaires complémentaires actifs dans l'UV-A et/ou l'UV-B, solubles dans au moins l'une des phases des compositions, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate.
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels solubles
le 3-(4'-sulfo)benzylidèn-bornan-2-one et ses sels solubles,
le 3-(4'méthylbenzylidène)-d,l-camphre,
le 3-benzylidène-d,l-camphre,
l'acide benzène 1,4-di(3-méthylidène-10-camphosulfonique) et ses sels solubles,
l'acide urocanique,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxobom-3-ylidèn)méthyl] benzyl]-acrylamide,
l'acide 1,4-bisbenzimidazolyl-phénylen-3,3',5,5'-tétrasulfonique et ses sels solubles.
les polyorganosiloxanes à fonction benzalmalonate
les polyorganosiloxanes à fonction benzotriazole tel que le Drometrizole Trisiloxane.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier la rémanence à l'eau, la stabilité, attachées intrinsèquement aux émulsions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour ies formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans l'utilisation d'une émulsion selon l'invention pour la fabrication de compositions cosmétiques pour la protection de la peau et/ou des cheveux contre le rayonnement ultraviolet, en particulier le rayonnement solaire.

Un autre objet de la présente invention réside dans l'utilisation d'un polymère associatif tel que défini précédemment pour la fabrication d'une émulsion cosmétique ou dermatologique photoprotectrice contenant au moins un filtre UV organique insoluble dans ladite émulsion, dans le but d'augmenter la résistance à l'eau de son pouvoir filtrant (rémanence à l'eau).

### EXEMPLES

| **COMPOSITION** | **EX 1** |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2g |
| Polydiméthylsiloxane (DOW CORNING 200 FLUID - DOW CORNING) | 0.5g |
| Benzoate d'alcools en C12/C15 (WITCONOL TN -WITCO) | 8g |
| Triéthanolamine | 0.5g |
| Copolymère vinylpyrrolidone/Eicosène (ANTARON V220 - ISP) | 2g |
| 4-tert-butyl-4'-méthoxy dibenzoylméthane) (PARSOL 1789-HOFFMANN LAROCHE) | 1.5g |
| Octocrylène (UVINUL N539-BASF) | 8g |
| Oxyde de titane (TITANIUM DIOXYDE MT-100 TV TAYCA) | 3g |
| Methylène bis-(tétraméthylbutyl hydroxyphényl benzotriazole) sous forme insoluble micronisée vendue sous le nom TINOSORB M par CIBA GEIGY- Taille moyenne de particule 0,15-0,2 µm | 4g |
| Propylène glycol | 4g |
| Glycérine | 4g |
| Acide benzène 1,4-di(3-méthylidène-10-camphosulfonique) (MEXORYL SX-CHIMEX) | 1g |
| EDTA | 0.1g |
| Acide polyacrylique (Synthalen K - 3V) | 0.3g |
| Triéthanolamine | qs pH :7 |
| Conservateurs | qs |
| Eau déminéralisée qsp | 100g |

| **COMPOSITION** | **EX 2** |
|---|---|
| Mélange mono /distéarate de glycérol / stéarate de polyéthylène glycol (100 OE) (ARLACEL 165 FL - ICI) | 1.5g |
| Alcool Cétylique | 1g |
| Acide stéarique d'huile de palme (STEARINE TP - STEARINERIE DUBOIS) | 2g |
| Polydiméthylsiloxane (DOW CORNING 200 FLUID - DOW CORNING) | 0.5g |
| Benzoate d'alcools en C12/C15 (WITCONOL TN -WITCO) | 8g |
| Triéthanolamine | 0.5g |
| 4-tert-butyl-4'-méthoxydibenzoylméthane) (PARSOL 1789-HOFFMANN LAROCHE) | 1g |
| Octocrylène (UVINUL N539-BASF) | 5g |
| Oxyde de titane (TITANIUM DIOXYDE MT-100 TV TAYCA) | 2g |
| Methylène bis-(tétraméthylbutyl hydroxyphényl benzotriazole) sous forme insoluble micronisée vendue sous le nom TINOSORB M par CIBA GEIGY- Taille moyenne de particule 0,15-0,2 µm | 5g |
| Propylène glycol | 4g |
| Glycérine | 4g |
| Acide 1,4-bis-benzimidazoyl-phénylèn-3,3',5,5'-tétrasulfonique-sel de sodium | 1g |
| Copolymère acide acrylique / Acrylate d'alkyle C₁₀/C₃₀ (PEMULEN TR1-GOODRICH) | 0.3g |
| Triéthanolamine | qs pH :7 |
| Conservateurs | qs |
| EDTA | 0.1 |
| Eau déminéralisée qsp | 100g |

| **COMPOSITION** | **EX 3** |
|---|---|
| Mélange mono /distéarate de glycerol / stéarate de polyéthylène glycol (100 OE) (ARLACEL 165 FL - ICI) | 1.5g |
| Alcool Cétylique | 1g |
| Acide stéarique d'huile de palme (STEARINE TP - STEARINERIE DUBOIS) | 2g |
| Poly diméthylsiloxane (DOW CORNING 200 FLUID - DOW CORNING) | 0.5g |
| Benzoate d'alcools en C12/C15 (WITCONOL TN -WITCO) | 6g |
| Triéthanolamine | 0.5g |
| Octylméthoxycinnamate (PARSOL MCX-HOFFMANN LAROCHE) | 5g |
| 2,4-bis{[4-2-éthyl-hexyloxy)]-2-hydroxy]-phényl}-6-(4-méthoxyphényl)-1,3,5-triazine | 3 |
| Methylène bis-(tétraméthylbutyl hydroxyphényl benzotriazole) sous forme insoluble micronisée vendue sous le nom TINOSORB M par CIBA GEIGY- Taille moyenne de particule 0,15-0,2 µm | 5g |
| Propylène glycol | 4g |
| Glycérine | 4g |
| Copolymère hexaméthyl diisocyanate/polyéthylèneglycol à terminaison alpha,oméga stéaryl polyoxyéthyléné (SER-AD FX1100 (SERVO-DELDEN) | 0.5g |
| Acide 1,4-bis-benzimidazoyl-phénylèn-3,3',5,5'-tétrasulfonique-sel de sodium | 0.5g |
| Triéthanolamine | qs pH :7 |
| Conservateurs | qs |
| Eau déminéralisée qsp | 100g |

## Revendications

1. Emulsion cosmétique ou dermatologique comportant au moins un système photoprotecteur capable de filtrer les rayons UV contenant au moins un filtre UV organique insoluble dans ladite émulsion, sous forme micronisée dont la taille moyenne des particules varie de 0,01 à 2 µm, **caractérisée par le fait qu'**elle comporte en plus au moins un polymère amphiphile comportant dans sa structure au moins une chaîne grasse et au moins une portion hydrophile ; ledit polymère étant différent d'un C₈-C₁₆alkylpolyglucoside.

2. Emulsion selon la revendication 1, où le polymère amphiphile est anionique, non-ionique, cationique ou amphotère.

3. Emulsion selon la revendication 2, où les polymères amphiphiles sont choisis parmi ceux comportant au moins un motif hydrophile et au moins un motif éther d'allyle à chaîne grasse.

4. Emulsion selon la revendication 3, où les polymères anioniques amphiphiles sont choisis parmi ceux dont le motif hydrophile est constitué par au moins un acide carboxylique vinylique et dont le motif éther d'allyle à chaîne grasse correspond au monomère de formule (I) suivante :
CH₂ = C(R')-CH₂ O Bₙ R (I)
dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone.

5. Emulsion selon la revendication 4, où le motif hydrophile est constitué par l'acide acrylique, l'acide méthacrylique ou leurs mélanges.

6. Emulsion selon la revendication 4 ou 5, où R' désigne H, n est égal à 10, et R désigne un radical stéaryle (C₁₈).

7. Emulsion selon l'une quelconque des revendications 3 à 6, où les polymères anioniques amphiphiles sont choisis parmi les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyle à chaîne grasse de formule (I), et de 0 à 1% en poids d'un agent réticulant.

8. Emulsion selon la revendication 7, où le polymère anionique amphiphile est choisi parmi les terpolymères réticulés d'acide méthacrylique, d'acrylate d'éthyle, de polyéthylèneglycol (10 OE) éther d'alcool stéarylique (Steareth 10).

9. Emulsion selon la revendication 2, où le polymère anionique amphiphile est choisi parmi les polymères anioniques comportant au moins un motif hydrophile de type acide carboxylique insaturé oléfinique, et au moins un motif hydrophobe exclusivement de type ester d'alkyl (C₁₀-C₃₀) d'acide carboxylique insaturé.

10. Emulsion selon la revendication 9, où le motif hydrophile de type acide carboxylique insaturé oléfinique correspond au monomère de formule (II) suivante : 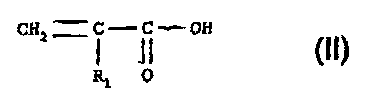 dans laquelle, R₁ désigne H ou CH₃ ou C₂H₅, c'est-à-dire des motifs acide acrylique, acide méthacrylique ou acide éthacrylique, et où le motif hydrophobe de type ester d'alkyl (C₁₀-C₃₀) d'acide carboxylique insaturé correspond au monomère de formule (III) suivante : 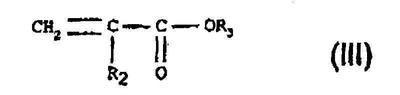 dans laquelle, R₂ désigne H ou CH₃ ou C₂H₅ ; R₃ désigne un radical alkyle en C₁₀-C₃₀, et de préférence en C₁₂-C₂₂.

11. Emulsion selon la revendication 10, où les esters d'alkyles (C₁₀-C₃₀) d'acides carboxyliques insaturés sont choisis parmi l'acrylate de lauryle, l'acrylate de stéaryle, l'acrylate de décyle, l'acrylate d'isodécyle et les méthacrylates correspondants.

12. Emulsion selon l'une quelconque des revendications 9 à 11, où le polymère anionique est formé à partir d'un mélange de monomères comprenant :
(i) essentiellement de l'acide acrylique,
(ii) un ester de formule (III) suivante : 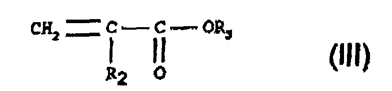 dans laquelle R₂ désigne H ou CH₃, R₃ désignant un radical alkyle ayant de 12 à 22 atomes de carbone,
(iii) et un agent réticulant.

13. Emulsion selon l'une quelconque des revendications 9 à 11, où le polymère anionique amphiphile est formé à partir d'un mélange de monomères comprenant de 95 à 60% en poids d'acide acrylique, 4 à 40% en poids d'acrylate d'alkyles en C₁₀-C₃₀, et 0 à 6% en poids de monomère polymérisable réticulant.

14. Emulsion selon l'une quelconque des revendications 9 à 11, où le polymère anionique amphiphile est formé à partir d'un mélange de monomères comprenant de 98 à 96% en poids d'acide acrylique, 1 à 4% en poids d'acrylate d'alkyle en C₁₀-C₃₀,et de 0,1 à 0,6% en poids de monomère polymérisable réticulant.

15. Emulsion selon la revendication 2, où les polymères amphiphiles non-ioniques sont choisis parmi :
(1) les celluloses modifiées par des groupements comportant au moins une chaîne grasse ;
(2) les hydroxypropylguars modifiés par des groupements comportant au moins une chaîne grasse ;
(3) les polyuréthanes polyéthers comportant dans leur chaîne, à la fois des séquences hydrophiles et des séquences hydrophobes qui peuvent être des enchaînements aliphatiques seuls et/ou des enchaînements cycloaliphatiques et/ou aromatiques ;
(4) ) les copolymères de vinyl pyrrolidone et de monomères hydrophobes à chaîne grasse ;
(5) les copolymères de méthacrylates ou d'acrylates d'alkyles en C₁-C₆ et de monomères amphiphiles comportant au moins une chaîne grasse ;
(6) les copolymères de méthacrylates ou d'acrylates hydrophiles et de monomères hydrophobes comportant au moins une chaîne grasse.

16. Emulsion selon la revendication 15, où les celluloses modifiées par des groupements comportant au moins une chaîne grasse sont choisies parmi :
- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂,
- les hydroxyéthylcelluloses modifiées par des groupes polyalkylène glycol éther d'alkyl phénol.

17. Emulsion selon la revendication 15, où les copolymères de vinyl pyrrolidone et de monomères hydrophobes à chaîne grasse sont choisis parmi :
- les copolymères vinylpyrrolidone / hexadécène ;
- les copolymères vinylpyrrolidone / eicosène.

18. Emulsion selon la revendication 15, où le copolymère de méthacrylate ou d'acrylate d'alkyle en C₁-C₆ et de monomère amphiphile comportant au moins une chaîne grasse est un copolymère acrylate de méthyle/acrylate de stéaryle oxyéthyléné.

19. Emulsion selon la revendication 15, où le copolymère de méthacrylate ou d'acrylate hydrophile et de monomère hydrophobe comportant au moins une chaîne grasse est un copolymère méthacrylate de polyéthylèneglycol/méthacrylate de lauryle.

20. Emulsion selon la revendication 15, où les polyuréthanes polyéthers amphiphiles non-ioniques comportent dans leur chaîne des séquences hydrophiles polyoxyéthylénées.

21. Emulsion selon la revendication 15, les polyuréthanes polyéthers amphiphiles non-ioniques comportent au moins deux chaînes hydrocarbonées hydrophobes, ayant de C₆ à C₃₀ atomes de carbone, séparées par une séquence hydrophile, les chaînes hydrocarbonées pouvant être des chaînes pendantes ou des chaînes en bout de séquence hydrophile.

22. Emulsion selon l'une quelconque des revendications 20 à 21, où les polyuréthanes polyéthers amphiphiles non-ioniques sont multiséquencés et de préférence tribloc.

23. Emulsion selon l'une quelconque des revendications 20 à 22, où les polyuréthanes polyéthers amphiphiles non-ioniques sont en greffons ou en étoile.

24. Emulsion selon l'une quelconque des revendications 20 à 23, où les polyuréthanes polyéthers amphiphiles non-ioniques sont choisis parmi des copolymères triblocs dont la séquence hydrophile est une chaîne polyoxyéthylénée comportant de 50 à 1000 groupements oxyéthylénés.

25. Emulsion selon la revendication 2, où les polymères amphiphiles cationiques sont choisis parmi les dérivés de cellulose quatemisée et les polyacrylates à groupements latéraux aminés.

26. Emulslon selon la revendication 25, où les dérivés de cellulose quatemisée sont choisis parmi :
- les celluloses quaternisées modifiées par des groupements comportant au moins une chaîne grasse, de préférence choisis parmi les groupes alkyle, arylalkyle, alkylaryle comportant au moins 8 atomes de carbone, de préférence de 8 à 30 atomes de carbone ; ou des mélanges de ceux-ci,
- les hydroxyéthylcelluloses quatemisées modifiées par des groupements comportant au moins une chaîne grasse, tels que les groupes alkyle, arylalkyle, alkylaryle comportant au moins 8 atomes de carbone, de préférence de 8 à 30 atomes de carbone ; ou des mélanges de ceux-ci.

27. Emulsion selon la revendication 25, où les polyacrylates à groupements latéraux aminés, quatemisés ou non, possèdent des groupements hydrophobes du type stéareth-20 (alcool stéarylique polyoxyéthyléné(20)).

28. Emulsion selon la revendication 2, où les polymères amphotères amphiphiles sont choisis parmi les polymères amphotères réticulés ou non réticulés, branchés ou non branchés, susceptibles d'être obtenus par la copolymérisation
1) d'au moins un monomère de formule (IVa) ou (IVb): 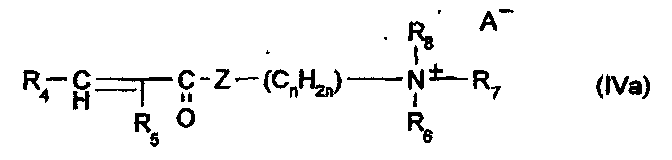 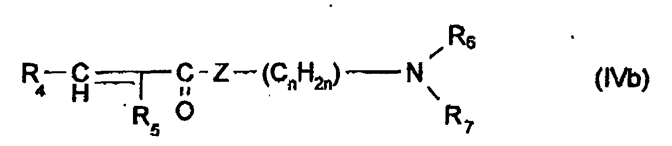 dans lesquelles R₄ et R₅, identiques ou différents, représentent un atome d'hydrogène ou un radical méthyle,
R₆, R₇ et R₈, identiques ou différents, représente un radical alkyle linéaire ou ramifié ayant de 1 à 30 atomes de carbone,
Z représente un groupe NH ou un atome d'oxygène,
n est un nombre entier de 2 à 5,
A⁻ est un anion issu d'un acide organique ou minéral, tel qu'un anion méthosulfate ou un halogénure tel que chlorure ou bromure.
2) d'au moins un monomère de formule (V) : 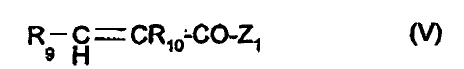 dans laquelle R₉ et R₁₀, identiques ou différents, représentent un atome d'hydrogène ou un radical méthyle;
Z₁ représente un groupe OH ou un groupe NHC(CH₃)₂CH₂SO₃H ;
3) d'au moins un monomère de formule (VI) : 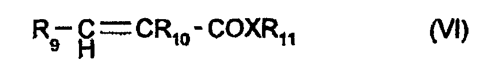 dans laquelle R₉ et R₁₀, identiques ou différents, représentent un atome d'hydrogène ou un radical méthyle, X désigne un atome d'oxygène ou d'azote et R₁₁ désigne un radical alkyle linéaire ou ramifié ayant de 1 à 30 atomes de carbone;
4) éventuellement au moins un agent de réticulation ou de branchement ; l'un au moins des monomères de formule (IVa), (IVb) ou (VI) comportant au moins une chaîne grasse ayant de 8 à 30 atomes de carbone et lesdits composés des monomères de formule (IVa), (IVb), (V) et (VI) pouvant être quatemisés par exemple par un halogénure d'alkyle en C₁-C₄ ou un sulfate de dialkyle en C₁-C₄.

29. Emulsion selon l'une quelconque des revendications 1 à 28, **caractérisée par le fait qu'**il s'agit d'une émulsion simple huile-dans-eau, d'une émulsion simple eau-dans-huile ou d'une émulsion complexe.

30. Emulsion selon la revendication 29, **caractérisée par le fait qu'**il s'agit d'une émulsion simple huile-dans-eau.

31. Emulsion selon la revendication 30, dans laquelle le ou les polymères amphiphiles jouent le rôle d'émulsionnant de la phase huileuse dans la phase aqueuse.

32. Emulsion selon la revendication 31, dans laquelle le ou les polymères amphiphiles sont présents en quantité allant de 0,1 à 20 % du poids total de la composition, de préférence en une quantité allant de 0,5 à 10 %.

33. Emulsion selon l'une quelconque des revendications 1 à 32, où le ou les filtres UV organiques insolubles sont choisis parmi les filtres UV organiques du type oxanilide, du type triazine, du type triazole, du type amide vinylique ou du type cinnamide.

34. Emulsion selon la revendication 33, où les filtres UV du type oxanilide, on peut citer ceux répondant à la structure : 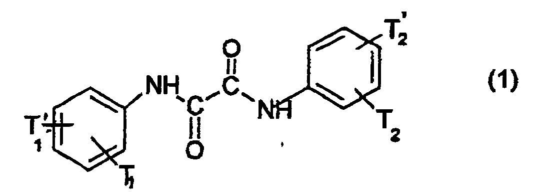 dans laquelle T₁, T'₁, T₂ et T'₂ désignent, identiques et différents, un radical alkyle en C₁-C₈ ou un radical alcoxy en C₁-C₈.

35. Emulsion selon la revendication 33, où les filtres UV du type triazine sont choisis parmi ceux répondant à la formule générale suivante : 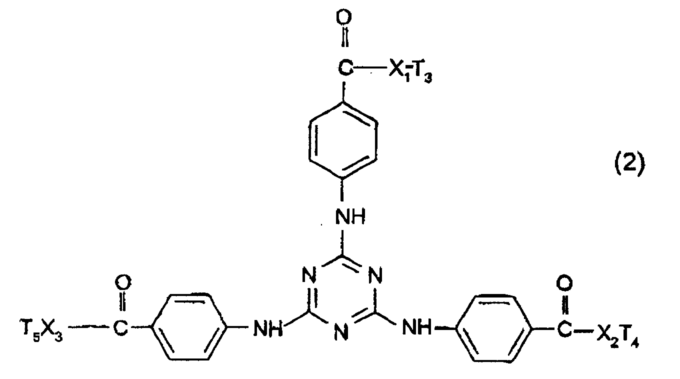 dans laquelle ;
- X₁, X₂ et X₃, identiques ou différents, représentent l'oxygène ou un radical -NZ-;
- les radicaux Z, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- T₃, T₄ et T₅, identiques ou différents, sont choisis parmi : l'hydrogène; un métal alcalin; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical polyoxyéthyléné comprenant de 1 à 6 unités d'oxyde d'éthylène et dont le groupe OH terminal est méthylé ; un radical de formule (3), (4) ou (5) suivantes : 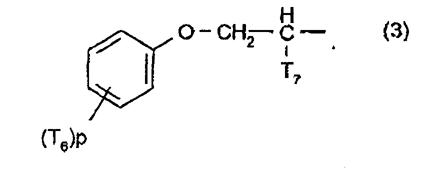 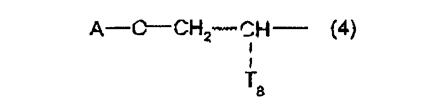 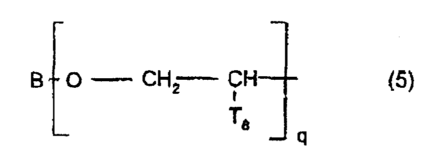 dans lesquelles
- T₆ est l'hydrogène ou un radical méthyle ;
- T₇ est un radical alkyle en C₁-C₉ ;
- p est un nombre entier allant de 0 à 3 ;
- q est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyle en C₁-C₄ ;
- T₈ est l'hydrogène ou un radical méthyle.

36. Emulsion selon la revendication 35, où le filtre UV du type triazine répond à la formule suivante : 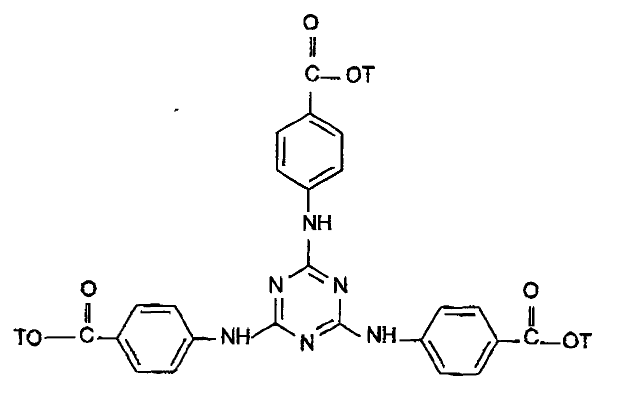 dans laquelle T désigne un radical 2-éthylhexyle ;

37. Emulsion selon la revendication 35, où le filtre UV du type triazine répond à la formule suivante : 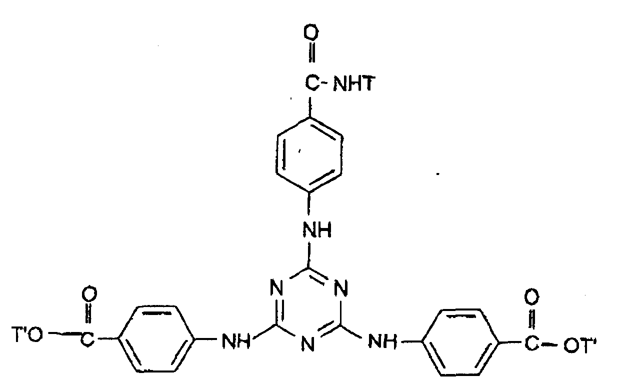 dans laquelle T' désigne un radical 2-éthylhexyle et T désigne un radical tert-butyle.

38. Emulsion selon la revendication 33, où les filtres UV du type triazine sont choisis parmi les dérivés insolubles de s-triazine portant des groupements benzalmalonates et/ou phenylcyanoacrylates.

39. Emulsion selon la revendication 38, où les filtres UV du type triazine sont choisis parmi les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(α-cyano-4-aminocinnamate d'éthyle)-s-triazine.

40. Emulsion selon la revendication 33, où les filtres UV du type triazine sont choisis parmi ceux répondant à la formule suivante : 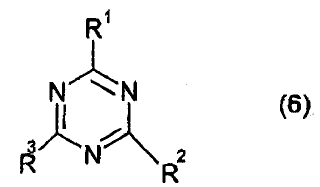 dans laquelle R¹, R², R³, indépendamment, sont phenyle, phenoxy, pyrrolo, dans lesquels les phenyle, phenoxy, pyrrolo sont éventuellement substitués par un, deux ou trois substituants choisis parmi OH, C₁-C₁₈alkyle ou alkoxy, C₁-C₁₈carboxyalkyle, C₅-C₈cycloalkyle, un groupe méthylidènecamphre, un groupe -(CH=CH)ₙ(CO)-OR⁴, avec R⁴ soit C₁-C₁₈alkyle soit cinnamyle, et n vaut 0 ou 1.

41. Emulsion selon la revendication 33, où les filtres UV du type triazine sont choisis parmi les dérivés insolubles de s-triazine portant des groupements benzotriazoles et/ou benzothiazoles.

42. Emulsion selon la revendication 41, où les filtres UV insolubles du type triazine sont choisis parmi
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

43. Emulsion selon la revendication 33, où les filtres UV organiques Insolubles du type triazole répondent à la formule (7) suivante : 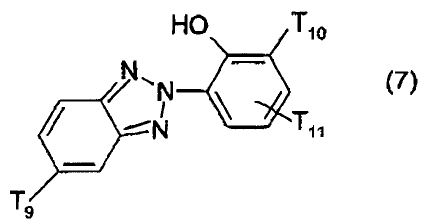 dans laquelle T₉ désigne un atome d'hydrogène ou un radical alkyle en C₁-C₁₈ ; T₁₀ et T₁₁, identiques ou différents, désignent un radical alkyle en C₁-C₁₈ éventuellement substitué par un phényle.

44. Emulsion selon la revendication 43, où le composé de formule (7) est choisi parmi les composés suivants :

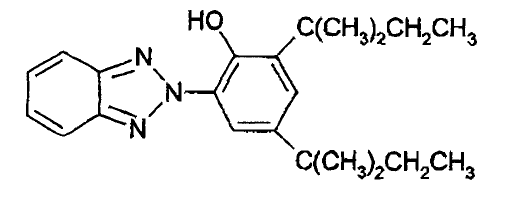

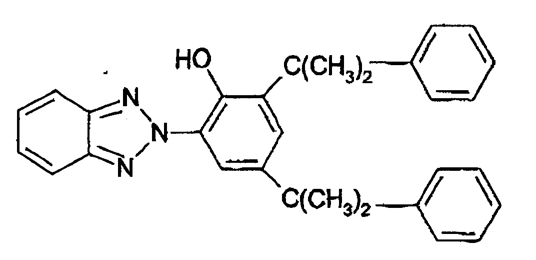

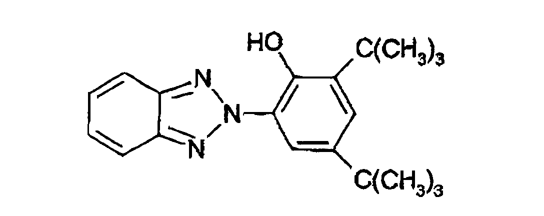

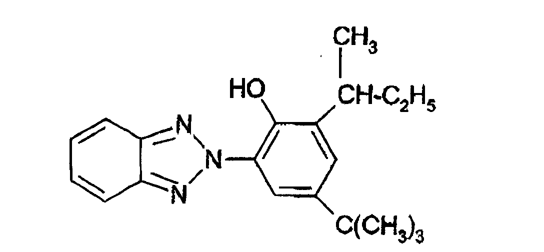

45. Emulsion selon la revendication 33, où le filtre UV insoluble est le [2 ,4'-dihydroxy-3-(2H-benzotriazol-2-yl)-5-(1,1,3,3-tétraméthylbutyl)-2'-n-octoxy-5'-benzoyl] diphénylméthane de structure :

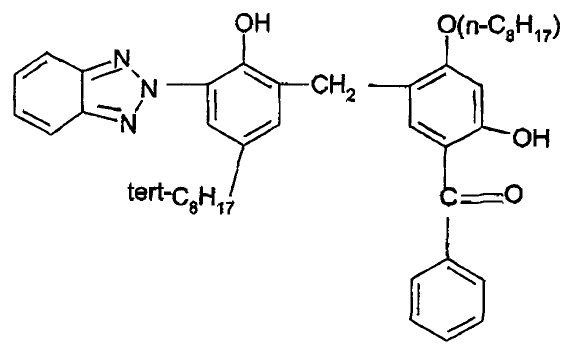

46. Emulsion selon la revendication 33, où les filtres UV organiques du type triazole sont choisis parmi les dérivés de méthylène bis-(hydroxyphényl benzotriazole) de structure suivante : 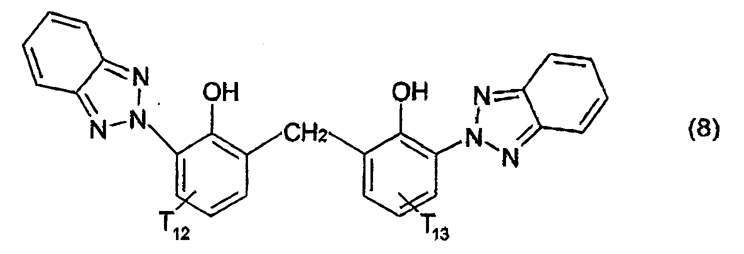 dans laquelle les radicaux T₁₂ et T₁₃, identiques ou différents, désignent un radical alkyle en C₁-C₁₈ pouvant être substitué par un ou plusieurs radicaux choisis parmi alkyle en C₁-C₄, cycloalkyle en C₅-C₁₂ ou un reste aryle.

47. Emulsion selon la revendication 46, où le composé de formule (8) est choisi dans le groupe constitué par les composés de structure suivante :

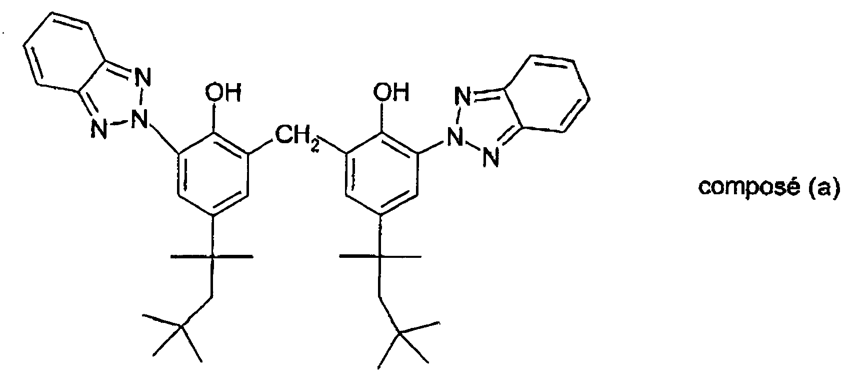

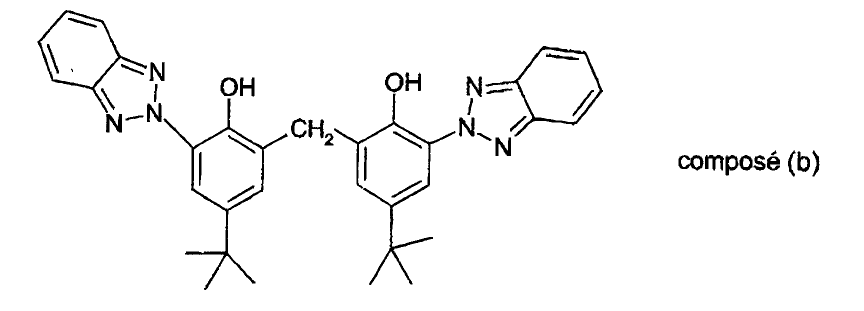

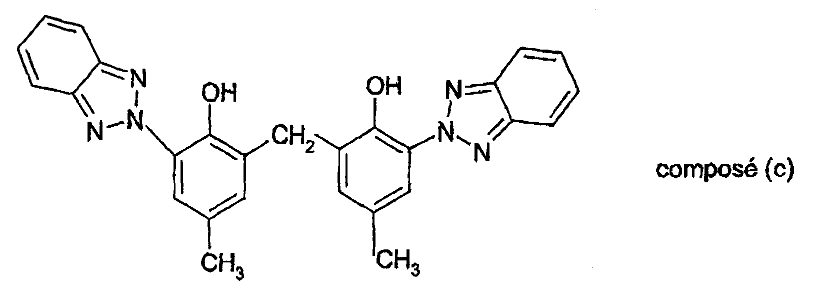

48. Emulsion selon la revendication 33, où les filtres organiques insolubles du type amide vinylique, répondent à la formule suivante :
T₁₄-(Y)r-C(=O)-C(T₁₅)=C(T₁₆)-N(T₁₇)(T₁₈) (9)
dans laquelle T₁₄ est un radical alkyle en C₁-C₁₈, de préférence en C₁-C₅ ou un groupe phényle éventuellement substitué par un, deux ou trois radicaux choisis parmi OH, alkyle en C₁-C₁₈, alcoxy en C₁-C₈, ou un groupe -C(=O)-OT₁₉ où T₁₉ est un alkyle en C₁-C₁₈ ; T₁₅, T₁₆, T₁₇ et T₁₈ identiques ou différents désignent un radical alkyle en C₁-C₁₈, de préférence en C₁-C₅ ou un atome d'hydrogène ; Y est N ou O et r vaut 0 ou 1.

49. Emulsion selon la revendication 48, où les composés de formule (9) sont choisis parmi :
- la 4-octylamino-3-pentèn-2-one ;
- l'éthyl-3-octylamino-2-buténoate ;
- la 3-octylamino-1-phényl-2-butèn-1-one
- la 3-dodecylamino-1-phenyl-2-buten-1-one.

50. Emulsion selon la revendication 33, où les filtres organiques insolubles du type cinnamamide ont comme formule : 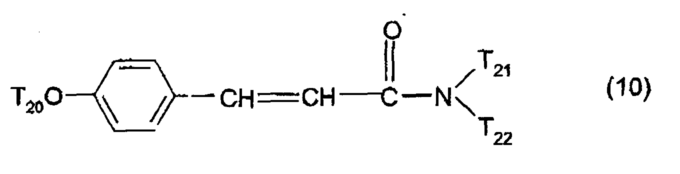 dans laquelle OT₂₀ est un radical hydroxy ou alcoxy en C₁-C₄, de préférence méthoxy ou éthoxy ; T₂₁ est hydrogène, alkyle en C₁-C₄, de préférence méthyle ou éthyle ; T₂₂ est un groupe -(CONH)s-phényle ou s vaut 0 ou 1 et le groupe phényle peut être substitué par un, deux ou trois groupes choisis parmi OH, alkyle en C₁-C₁₈, alcoxy en C₁-C₈, ou un groupe -C(=O)-OT₂₃ où T₂₃ est un alkyle en C₁-C₁₈ et plus préférentiellement T₂₃ est un groupe phényle, 4-méthoxyphényle ou phénylaminocarbonyle.

51. Emulsion selon la revendication 33, où le filtre UV insoluble est un dimère cinnamamide.

52. Emulsion selon la revendication 51, où le filtre UV insoluble est le composé de structure :

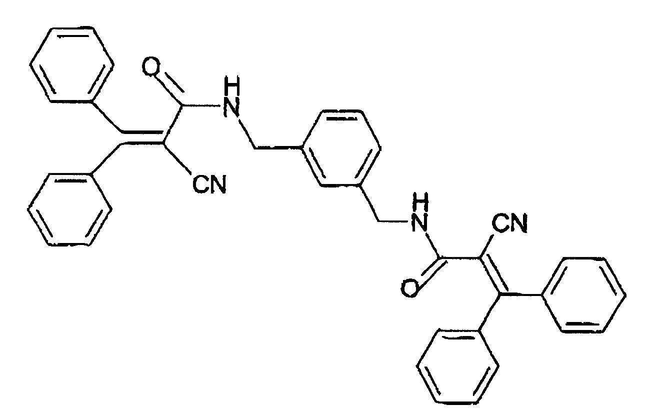

53. Emulsion selon l'une quelconque des revendications 1 à 32, où les filtres UV insolubles sont des sels de métaux polyvalents de filtres UV organiques sulfoniques ou carboxyliques.

54. Emulsion selon la revendication 33, où les filtres UV insolubles sont choisis parmi les sels de métaux polyvalents de dérivés sulfonés de benzylidène camphre ; les sels de métaux polyvalents de dérivés sulfonés de benzimidazole ; les sels de métaux polyvalents de dérivés d'acide cinnamique.

55. Emulsion selon l'une quelconque des revendications 1 à 32, où les filtres UV insolubles sont des complexes de métaux polyvalents ou d'ammonium ou d'ammonium substitué de filtres organiques UV-A et/ou UV-B.

56. Emulsion selon l'une quelconque des revendications 1 à 55, où le ou les filtres UV insolubles sous forme insoluble micronisée a une taille moyenne de particule allant de 0,02 à 1,5 µm.

57. Emulsion selon la revendication 56, où le ou les filtres UV insolubles sous forme insoluble micronisée a une taille moyenne de particule allant de 0,03 à 1,0 µm.

58. Emulsion selon l'une quelconque des revendications 1 à 57, **caractérisé par le fait que** le ou les filtres UV insolubles sous forme micronisée sont susceptibles d'être obtenus par un procédé de broyage du filtre organique insoluble sous forme de particules de taille grossière en présence d'un tensio-actif.

59. Emulsion selon la revendication 58, où le tensio-actif est choisi parmi les alkylpolyglucosides de structure CₙH₂ₙ₊₁O(C₆H₁₀O₅)ₓH dans laquelle n est un entier de 8 à 16 et x est le degré moyen de polymérisation de l'unité (C₆H₁₀O₅) et varie de 1,4 à 1,6.

60. Emulsion selon l'une quelconque des revendications 58 à 59, **caractérisé par le fait que** le tensio-actif est utilisé à une concentration allant de 1 à 50% en poids par rapport au filtre UV organique insoluble dans sa forme micronisée.

61. Composition cosmétique ou dermatologique, destinée à la photoprotection de la peau et/ou des cheveux, **caractérisée par le fait qu'**elle comprend au moins dans un support cosmétiquement acceptable une émulsion telle que définie dans l'une quelconque des revendications 1 à 60.

62. Composition selon la revendication 61, **caractérisée par le fait qu'**elle comprend en outre un ou plusieurs filtres organiques complémentaires actifs dans l'UV-A et/ou UV-B, solubles dans l'une des phase de la composition.

63. Composition selon la revendication 62, **caractérisée par le fait que** lesdits filtres organiques complémentaires sont choisis parmi les dérivés cinnamiques ; les dérivés salicyliques, les dérivés du camphre; les dérivés de triazine ; les dérivés de dibenzoylméthane ; les dérivés de la benzophénone ; les dérivés de β,β'-diphénylacrylate ; les dérivés de benzimidazole ; les dimères dérivés d'α-alkylstyrène ; les dérivés bis-benzoazolyle ; les dérivés de l'acide p-aminobenzoïque ; les polymères filtres et silicones filtres.

64. Composition selon l'une quelconque des revendications précédentes, **caractérisée par le fait qu'**elle comprend en outre des pigments ou des nanopigments d'oxydes métalliques, enrobés ou non.

65. Composition selon la revendication 64, **caractérisée par le fait que** lesdits pigments ou nanopigments sont choisis parmi les oxydes de titane, de zinc, de fer, de zirconium, de cérium et leurs mélanges, enrobés ou non.

66. Composition selon l'une quelconque des revendications 61 à 66, **caractérisée par le fait qu'**elle comprend en outre au moins un agent de bronzage et/ou de brunissage artificiel de la peau.

67. Composition selon l'une quelconque des revendications 61 à 66, **caractérisée par le fait qu'**elle comprend en outre au moins un adjuvant choisi parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants.

68. Composition selon l'une quelconque des revendications 61 à 67, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

69. Composition selon l'une quelconque des revendications 61 à 67, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

70. Composition selon l'une quelconque des revendications 61 à 67, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

71. Utilisation de l'émulsion définie dans l'une quelconque des revendications 1 à 60 pour la fabrication de compositions cosmétiques pour la protection de la peau et/ou des cheveux contre le rayonnement ultraviolet, en particulier le rayonnement solaire.

72. Utilisation d'un polymère amphiphile comportant dans sa structure au moins une chaîne grasse et au moins une portion hydrophile tel que défini dans l'une quelconque des revendications précédentes pour la fabrication d'une émulsion cosmétique ou dermatologique photoprotectrice contenant au moins un filtre organique UV insoluble dans l'émulsion, dans le but d'augmenter la résistance à l'eau de son pouvoir filtrant (rémanence à l'eau).

## Claims

1. Cosmetic or dermatological emulsion comprising at least one photoprotective system capable of screening out UV rays, containing at least one organic UV-screening agent insoluble in the said emulsion, in micronized form, in which the mean size of the particles varies from 0.01 to 2 µm, **characterized in that** it comprises in addition at least one amphiphilic polymer whose structure contains at least one fatty chain and at least one hydrophilic portion; the said polymer being different from a C₈-C₁₆ alkyl polyglucoside.

2. Emulsion according to Claim 1, where the amphiphilic polymer is anionic, nonionic, cationic or amphoteric.

3. Emulsion according to Claim 2, where the amphiphilic polymers are chosen from those comprising at least one hydrophilic unit and at least one allyl ether unit containing a fatty chain.

4. Emulsion according to Claim 3, where the amphiphilic anionic polymers are chosen from those whose hydrophilic unit consists of at least one vinylcarboxylic acid and whose allyl ether unit containing a fatty chain corresponds to the monomer having the following formula (I):
CH₂ = C(R')-CH₂ O Bₙ R (I)
in which R' denotes H or CH₃, B denotes the ethyleneoxy radical, n is zero or denotes an integer from 1 to 100, R denotes a hydrocarbon radical chosen from the alkyl, arylalkyl, aryl, alkylaryl or cycloalkyl radicals, comprising 8 to 30 carbon atoms, preferably 10 to 24, and still more particularly from 12 to 18 carbon atoms.

5. Emulsion according to Claim 4, where the hydrophilic unit consists of acrylic acid, methacrylic acid or mixtures thereof.

6. Emulsion according to Claim 4 or 5, where R' denotes H, n is equal to 10, and R denotes a stearyl (C₁₈) radical.

7. Emulsion according to any one of Claims 3 to 6, where the amphiphilic anionic polymers are chosen from the polymers formed from 20 to 60% by weight of acrylic acid and/or methacrylic acid, from 5 to 60% by weight of lower alkyl (meth)acrylates, from 2 to 50% by weight of allyl ether containing a fatty chain of formula (I), and from 0 to 1% by weight of a crosslinking agent.

8. Emulsion according to Claim 7, where the amphiphilic anionic polymer is chosen from the crosslinked terpolymers of methacrylic acid, ethyl acrylate, polyethylene glycol (10 EO) stearyl alcohol ether (steareth 10).

9. Emulsion according to Claim 2, where the amphiphilic anionic polymer is chosen from the anionic polymers comprising at least one hydrophilic unit of the olefinic unsaturated carboxylic acid type, and at least one hydrophobic unit exclusively of the (C₁₀-C₃₀)alkyl ester of unsaturated carboxylic acid type.

10. Emulsion according to Claim 9, where the hydrophilic unit of the olefinic unsaturated carboxylic acid type corresponds to the monomer having the following formula (II): 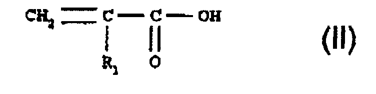 in which R₁ denotes H or CH₃ or C₂H₅, that is to say acrylic acid, methacrylic acid or ethacrylic acid units, and where the hydrophobic unit of the (C₁₀-C₃₀)alkyl ester of unsaturated carboxylic acid type corresponds to the monomer having the following formula (III): 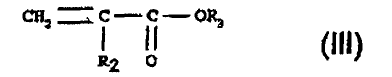 in which R₂ denotes H or CH₃ or C₂H₅; R₃ denotes a C₁₀-C₃₀, and preferably C₁₂-C₂₂, alkyl radical.

11. Emulsion according to Claim 10, where the (C₁₀-C₃₀)alkyl esters of unsaturated carboxylic acids are chosen from lauryl acrylate, stearyl acrylate, decyl acrylate, isodecyl acrylate and the corresponding methacrylates.

12. Emulsion according to any one of Claims 9 to 11, where the anionic polymer is formed from a mixture of monomers comprising:
(i) essentially acrylic acid,
(ii) an ester having the following formula (III): 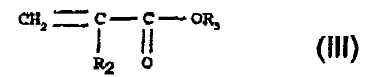 in which R₂ denotes H or CH₃, R₃ denoting an alkyl radical having from 12 to 22 carbon atoms,
(iii) and a crosslinking agent.

13. Emulsion according to any one of Claims 9 to 11, where the amphiphilic anionic polymer is formed from a mixture of monomers comprising from 95 to 60% by weight of acrylic acid, 4 to 40% by weight of C₁₀-C₃₀ alkyl acrylate and 0 to 6% by weight of crosslinking polymerizable monomer.

14. Emulsion according to any one of Claims 9 to 11, where the amphiphilic anionic polymer is formed from a mixture of monomers comprising from 98 to 96% by weight of acrylic acid, 1 to 4% by weight of C₁₀-C₃₀ alkyl acrylate and 0.1 to 0.6% by weight of crosslinking polymerizable monomer.

15. Emulsion according to Claim 2, where the nonionic amphiphilic polymers are chosen from:
(1) the celluloses modified by groups comprising at least one fatty chain;
(2) the hydroxypropylguars modified by groups comprising at least one fatty chain;
(3) the polyether-polyurethanes comprising in their chain both hydrophilic sequences and hydrophobic sequences which may be aliphatic chains alone and/or cycloaliphatic and/or aromatic chains;
(4) the copolymers of vinylpyrrolidone and of hydrophobic monomers having a fatty chain;
(5) the copolymers of C₁-C₆ alkyl methacrylates or acrylates and of amphiphilic monomers comprising at least one fatty chain;
(6) the copolymers of hydrophilic methacrylates or acrylates and of hydrophobic monomers comprising at least one fatty chain.

16. Emulsion according to Claim 15, where the celluloses modified by groups comprising at least one fatty chain are chosen from:
- hydroxyethylcelluloses modified by groups comprising at least one fatty chain such as alkyl, arylalkyl or alkylaryl groups, or mixtures thereof, and in which the alkyl groups are preferably C₈-C₂₂,
- the hydroxyethylcelluloses modified by polyalkylene glycol ether of alkylphenol groups.

17. Emulsion according to Claim 15, where the copolymers of vinylpyrrolidone and of hydrophobic monomers containing a fatty chain are chosen from:
- the vinylpyrrolidone/hexadecene copolymers;
- the vinylpyrrolidone/eicosene copolymers.

18. Emulsion according to Claim 15, where the copolymer of C₁-C₆ alkyl methacrylate or acrylate and of amphiphilic monomer comprising at least one fatty chain is an oxyethylenated stearyl acrylate/methyl acrylate copolymer.

19. Emulsion according to Claim 15, where the copolymer of hydrophilic methacrylate or acrylate and of hydrophobic monomer comprising at least one fatty chain is a polyethylene glycol methacrylate/ lauryl methacrylate copolymer.

20. Emulsion according to Claim 15, where the nonionic amphiphilic polyether-polyurethanes comprise polyoxyethylenated hydrophilic sequences in their chain.

21. Emulsion according to Claim 15, where the nonionic amphiphilic polyether-polyurethanes comprise at least two hydrophobic hydrocarbon chains, having from C₆ to C₃₀ carbon atoms, separated by a hydrophilic sequence, it being possible for the hydrocarbon chains to be pendent chains or chains at the end of a hydrophilic sequence.

22. Emulsion according to any one of Claims 20 to 21, where the nonionic amphiphilic polyether-polyurethanes are polyblocks and preferably triblocks.

23. Emulsion according to any one of Claims 20 to 22, where the nonionic amphiphilic polyether-polyurethanes are in the form of graft units or are star-shaped.

24. Emulsion according to any one of Claims 20 to 23, where the nonionic amphiphilic polyether-polyurethanes are chosen from triblock copolymers whose hydrophilic sequence is a polyoxyethylenated chain comprising from 50 to 1000 oxyethylenated groups.

25. Emulsion according to Claim 2, where the cationic amphiphilic polymers are chosen from quaternized cellulose derivatives and polyacrylates with amine-containing side groups.

26. Emulsion according to Claim 25, where the quaternized cellulose derivatives are chosen from:
- the quaternized celluloses modified by groups comprising at least one fatty chain, preferably chosen from the alkyl, arylalkyl or alkylaryl groups comprising at least 8 carbon atoms, preferably from 8 to 30 carbon atoms; or mixtures thereof,
- the quaternized hydroxyethylcelluloses modified by groups comprising at least one fatty chain, such as the alkyl, arylalkyl or alkylaryl groups comprising at least 8 carbon atoms, preferably from 8 to 30 carbon atoms; or mixtures thereof.

27. Emulsion according to Claim 25, where the polyacrylates with amine-containing side groups, quaternized or otherwise, possess hydrophobic groups of the steareth-20 type (polyoxyethylenated (20) stearyl alcohol).

28. Emulsion according to Claim 2, where the amphiphilic amphoteric polymers are chosen from crosslinked or non-crosslinked, branched or unbranched amphoteric polymers which can be obtained by copolymerization
1) of at least one monomer of formula (IVa) or (IVb): 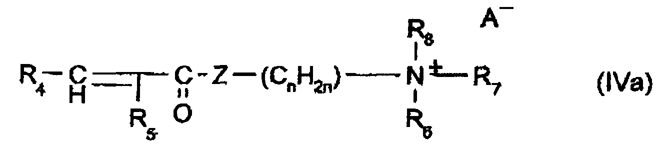 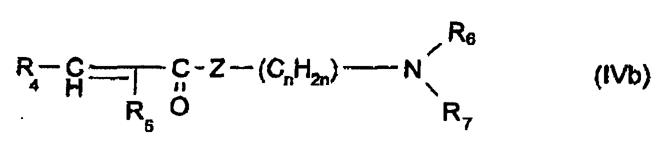 in which R₄ and R₅, which are identical or different, represent a hydrogen atom or a methyl radical,
R₆, R₇ and R₈, which are identical or different, represent a linear or branched alkyl radical having from 1 to 30 carbon atoms,
Z represents an NH group or an oxygen atom,
n is an integer from 2 to 5,
A⁻ is an anion derived from an organic or inorganic acid, such as a methosulphate anion or a halide such as chloride or bromide,
2) of at least one monomer of formula (V): 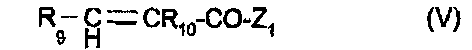 in which R₉ and R₁₀, which are identical or different, represent a hydrogen atom or a methyl radical;
Z₁ represents an OH group or a group NHC(CH₃)₂CH₂SO₃H;
3) of at least one monomer of formula (VI): 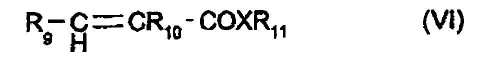 in which R₉ and R₁₀, which are identical or different, represent a hydrogen atom or a methyl radical, X denotes an oxygen or nitrogen atom and R₁₁ denotes a linear or branched alkyl radical having from 1 to 30 carbon atoms;
4) optionally at least one crosslinking or branching agent; at least one of the monomers of formula (IVa), (IVb) or (VI) comprising at least one fatty chain having from 8 to 30 carbon atoms and it being possible for the said compounds of the monomers of formula (IVa), (IVb), (V) and (VI) to be quaternized for example by a C₁-C₄ alkyl halide or a C₁-C₄ dialkyl sulphate.

29. Emulsion according to any one of Claims 1 to 28, **characterized in that** it is a simple oil-in-water emulsion, a simple water-in-oil emulsion or a complex emulsion.

30. Emulsion according to Claim 29, **characterized in that** it is a simple oil-in-water emulsion.

31. Emulsion according to Claim 30, in which the amphiphilic polymer(s) play the role of emulsifier of the oily phase in the aqueous phase.

32. Emulsion according to Claim 31, in which the amphiphilic polymer(s) are present in a quantity ranging from 0.1 to 20% of the total weight of the composition, preferably in a quantity ranging from 0.5 to 10%.

33. Emulsion according to any one of Claims 1 to 32, where the insoluble organic UV-screening agent(s) are chosen from the organic UV-screening agents of the oxanilide type, of the triazine type, of the triazole type, of the vinylamide type and of the cinnamide type.

34. Emulsion according to Claim 33, where among the UV-screening agents of the oxanilide type, there may be mentioned those corresponding to the structure: 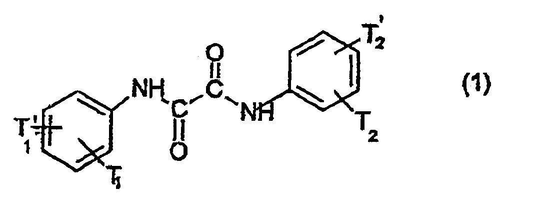 in which T₁, T'₁, T₂ and T'₂, which are identical or different, denote a C₁-C₈ alkyl radical or a C₁-C₈ alkoxy radical.

35. Emulsion according to Claim 33, where the UV-screening agents of the triazine type are chosen from those corresponding to the following general formula: 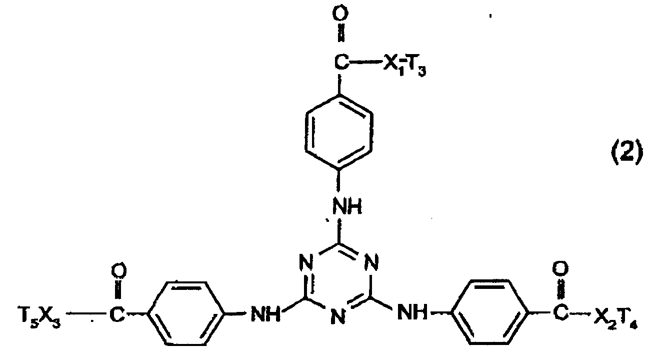 in which:
- X₁, X₂ and X₃, which are identical or different, represent oxygen or a radical -NZ-;
- the radicals Z, which are identical or different, denote hydrogen or a linear or branched C₁-C₁₈ alkyl radical, a C₅-C₁₂ cycloalkyl radical which may be substituted with one or more C₁-C₄ alkyl radicals;
- T₃, T₄ and T₅, which are identical or different, are chosen from hydrogen; an alkali metal; an ammonium radical which is optionally substituted with one or more alkyl or hydroxyalkyl radicals; a linear or branched C₁-C₁₈ alkyl radical; a C₅-C₁₂ cycloalkyl radical which is optionally substituted with one or more C₁-C₄ alkyl radicals; a polyoxyethylenated radical comprising from 1 to 6 ethylene oxide units and whose terminal OH group is methylated; a radical of the following formula (3), (4) or (5): 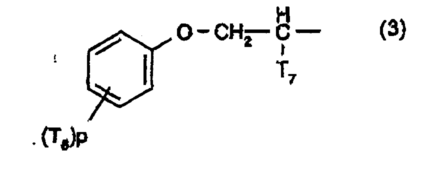 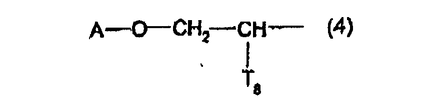 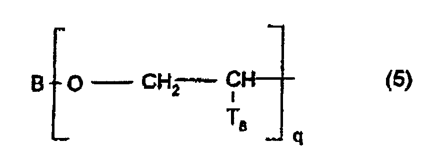 in which:
- T₆ is hydrogen or a methyl radical;
- T₇ is a C₁-C₉ alkyl radical;
- p is an integer ranging from 0 to 3;
- q is an integer ranging from 1 to 10;
- A is a C₄-C₈ alkyl radical or a C₅-C₈ cycloalkyl radical;
- B is chosen from a linear or branched C₁-C₈ alkyl radical; a C₅-C₈ cycloalkyl radical; an aryl radical which is optionally substituted with one or more C₁-C₄ alkyl radicals;
- T₈ is hydrogen or a methyl radical.

36. Emulsion according to Claim 35, where the UV-screening agent of the triazine type corresponds to the following formula: 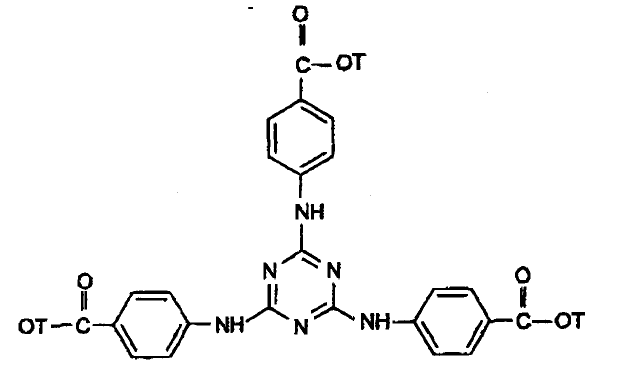 in which T denotes a 2-ethylhexyl radical.

37. Emulsion according to Claim 35, where the UV-screening agent of the triazine type corresponds to the following formula: 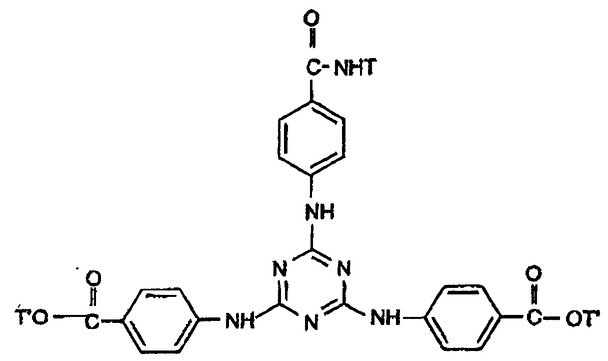 in which T' denotes a 2-ethylhexyl radical and T denotes a tert-butyl radical.

38. Emulsion according to Claim 33, where the UV-screening agents of the triazine type are chosen from the insoluble derivatives of s-triazine carrying benzalmalonate and/or phenylcyanoacrylate groups.

39. Emulsion according to Claim 38, where the UV-screening agents of the triazine type are chosen from the following compounds:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

40. Emulsion according to Claim 33, where the UV-screening agents of the triazine type are chosen from those corresponding to the following formula: 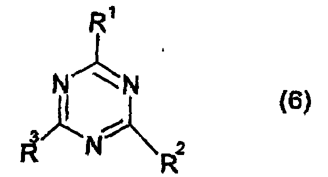 in which R¹, R², R³ are independently phenyl, phenoxy, pyrrolo, in which the phenyl, phenoxy and pyrrolo are optionally substituted with one, two or three substituents chosen from OH, C₁-C₁₈ alkyl or alkoxy, C₁-C₁₈ carboxyalkyl, C₅-C₈ cycloalkyl, a methylidenecamphor group, a group -(CH=CH)ₙ(CO)-OR⁴, with R⁴ either C₁-C₁₈ alkyl or cinnamyl, and n is equal to 0 or 1.

41. Emulsion according to Claim 33, where the UV-screening agents of the triazine type are chosen from the insoluble derivatives of s-triazine carrying benzotriazole and/or benzothiazole groups.

42. Emulsion according to Claim 41, where the insoluble UV-screening agents of the triazine type are chosen from:
- 2,4,6-tris[(3'-benzotriazol-2-yl-2'-hydroxy-5'-methyl)phenylamino]-s-triazine,
- 2,4,6-tris[(3'-benzotriazol-2-yl-2'-hydroxy-5'-ter-octyl)phenylamino]-s-triazine.

43. Emulsion according to Claim 33, where the insoluble organic UV-screening agents of the triazole type correspond to the following formula (7): 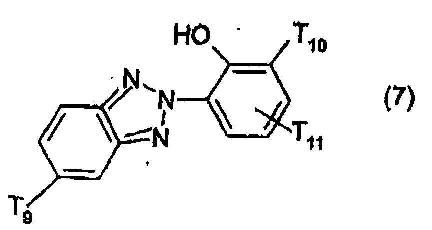 in which T₉ denotes a hydrogen atom or a C₁-C₁₈ alkyl radical; T₁₀ and T₁₁, which are identical or different, denote a C₁-C₁₈ alkyl radical which is optionally substituted with a phenyl.

44. Emulsion according to Claim 43, where the compound of formula (7) is chosen from the following compounds:

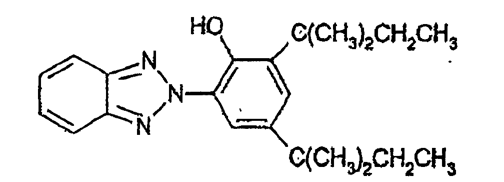

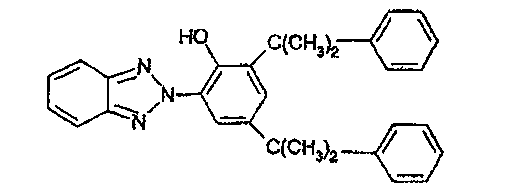

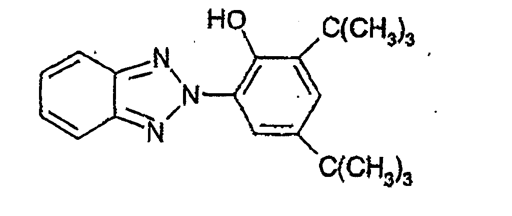

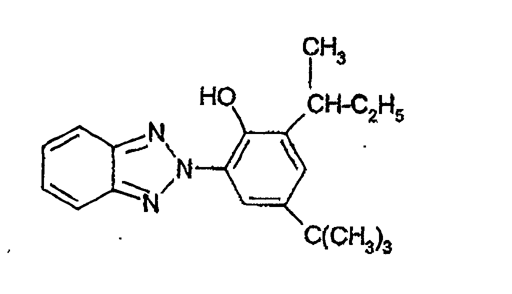

45. Emulsion according to Claim 33, where the insoluble UV-screening agent is [2,4'-dihydroxy-3-(2H-benzotriazol-2-yl)-5-(1,1,3,3-tetramethylbutyl)-2'-n-octoxy-5'-benzoyl]diphenylmethane having the structure:

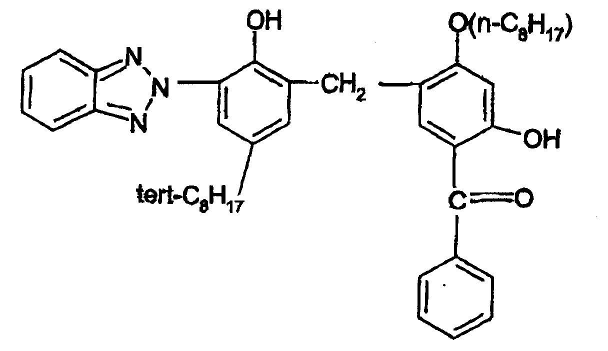

46. Emulsion according to Claim 33, where the organic UV-screening agents of the triazole type are chosen from the methylenebis(hydroxyphenylbenzotriazole) derivatives having the following structure: 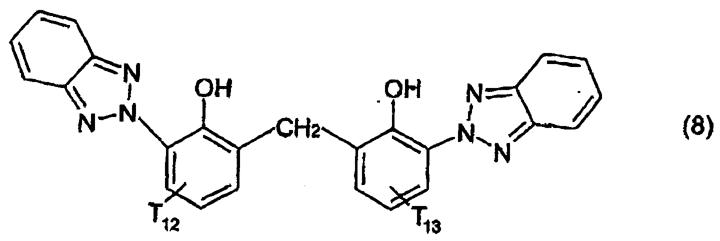 in which the radicals T₁₂ and T₁₃, which are identical or different, denote a C₁-C₁₈ alkyl radical which may be substituted with one or more radicals chosen from a C₁-C₄ alkyl, a C₅-C₁₂ cycloalkyl or an aryl residue.

47. Emulsion according to Claim 46, where the compound of formula (8) is chosen from the group consisting of the compounds having the following structure:

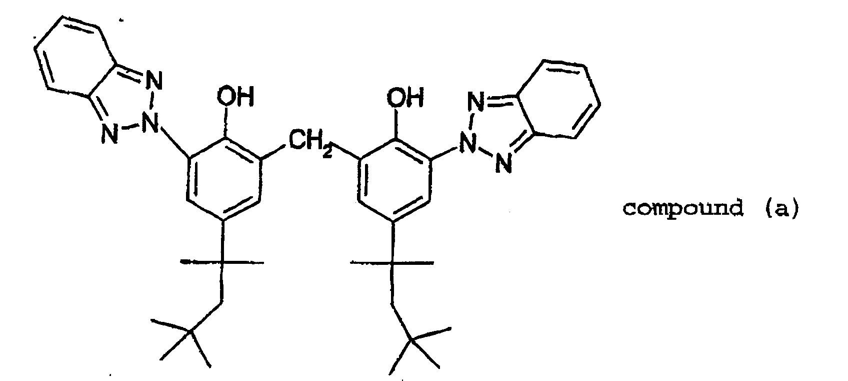

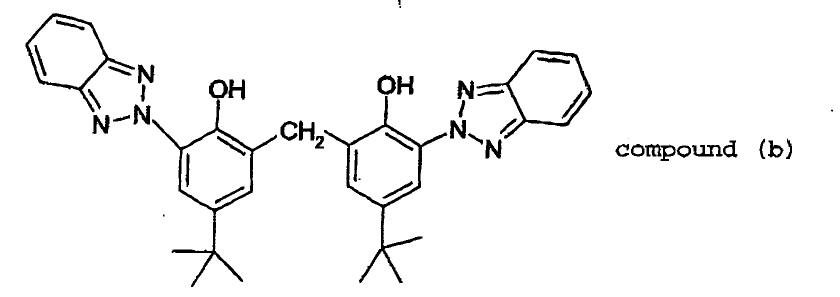

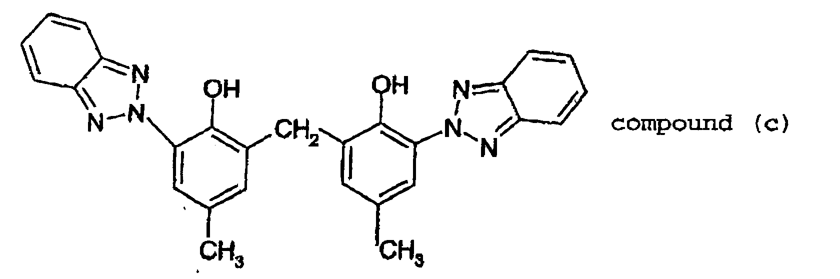

48. Emulsion according to Claim 33, where the insoluble organic screening agents of the vinylamide type correspond to the following formula:
T₁₄- (Y)r-C(=O)-C(T₁₅)=C(T₁₆)-N(T₁₇) (T₁₈) (9)
in which T₁₄ is a C₁-C₁₈, preferably C₁-C₅, alkyl radical or a phenyl group which is optionally substituted with one, two or three radicals chosen from OH, C₁-C₁₈ alkyl, C₁-C₈ alkoxy, or a group -C(=O)-OT₁₉ where T₁₉ is a C₁-C₁₈ alkyl; T₁₅, T₁₆, T₁₇ and T₁₈, which are identical or different, denote a C₁-C₁₈, preferably C₁-C₅, alkyl radical or a hydrogen atom; Y is N or O and r is equal to 0 or 1.

49. Emulsion according to Claim 48, where the compounds of formula (9) are chosen from:
- 4-octylamino-3-penten-2-one;
- ethyl 3-octylamino-2-butenoate;
- 3-octylamino-1-phenyl-2-buten-1-one;
- 3-dodecylamino-1-phenyl-2-buten-1-one.

50. Emulsion according to Claim 33, where the insoluble organic screening agents of the cinnamamide type have as formula 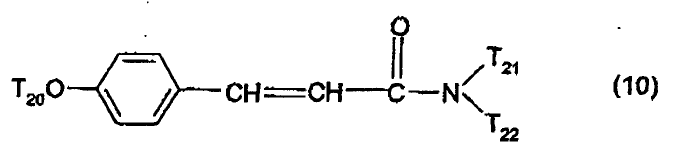 in which OT₂₀ is a hydroxyl or C₁-C₄ alkoxy, preferably methoxy or ethoxy, radical; T₂₁ is hydrogen, C₁-C₄ alkyl, preferably methyl or ethyl; T₂₂ is a group -(CONH)s-phenyl where s is equal to 0 or 1 and the phenyl group may be substituted with one, two or three groups chosen from OH, C₁-C₁₈ alkyl, C₁-C₈ alkoxy, or a group -C(=O)-OT₂₃ where T₂₃ is a C₁-C₁₈ alkyl and more preferably T₂₃ is a phenyl, 4-methoxyphenyl or phenylaminocarbonyl group.

51. Emulsion according to Claim 33, where the insoluble UV-screening agent is a cinnamamide dimer.

52. Emulsion according to Claim 51, where the insoluble UV-screening agent is the compound having the structure:

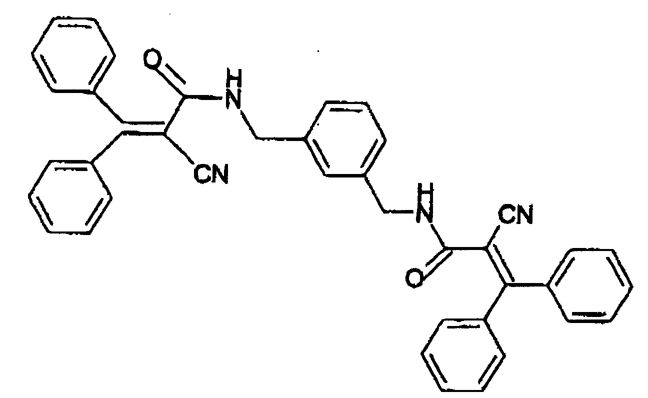

53. Emulsion according to any one of Claims 1 to 32, where the insoluble UV-screening agents are polyvalent metal salts of sulphonic or carboxylic organic UV-screening agents.

54. Emulsion according to Claim 33, where the insoluble UV-screening agents are chosen from the polyvalent metal salts of sulphonated derivatives of benzylidenecamphor; the polyvalent metal salts of sulphonated derivatives of benzimidazole; the polyvalent metal salts of derivatives of cinnamic acid.

55. Emulsion according to any one of Claims 1 to 32, where the insoluble UV-screening agents are complexes of polyvalent metals or of ammonium or of ammonium substituted with organic UV-A and/or UV-B screening agents.

56. Emulsion according to any one of Claims 1 to 55, where the insoluble UV-screening agent(s) in micronized insoluble form has a mean particle size ranging from 0.02 to 1.5 µm.

57. Emulsion according to Claim 56, where the insoluble UV-screening agent(s) in micronized insoluble form has a mean particle size ranging from 0.03 to 1.0 µm.

58. Emulsion according to any one of Claims 1 to 57, **characterized in that** the insoluble UV-screening agent (s) in micronized form can be obtained by a method of grinding the insoluble organic screening agent in the form of particles of coarse size in the presence of a surfactant.

59. Emulsion according to Claim 58, where the surfactant is chosen from alkyl polyglucosides having the structure CₙH₂ₙ₊₁O(C₆H₁₀O₅)ₓH in which n is an integer from 8 to 16 and x is the average degree of polymerization of the unit (C₆H₁₀O₅) and varies from 1.4 to 1.6.

60. Emulsion according to either of Claims 58 and 59, **characterized in that** the surfactant is used at a concentration ranging from 1 to 50% by weight relative to the insoluble organic UV-screening agent in its micronized form.

61. Cosmetic or dermatological composition intended for the photoprotection of the skin and/or of the hair, **characterized in that** it comprises at least in a cosmetically acceptable carrier an emulsion as defined in any one of Claims 1 to 60.

62. Composition according to Claim 61, **characterized in that** it comprises, in addition, one or more additional organic screening agents active in UV-A and/or UV-B, soluble in one of the phases of the composition.

63. Composition according to Claim 62, **characterized in that** the said additional organic screening agents are chosen from the cinnamic derivatives; the salicylic derivatives; the camphor derivatives; the triazine derivatives; the dibenzoylmethane derivatives; the benzophenone derivatives; the β,β'-diphenyl acrylate derivatives; the benzimidazole derivatives; the dimers derived from α-alkylstyrene; the bisbenzoazolyl derivatives; the p-aminobenzoic acid derivatives; the polymer screening agents and silicone screening agents.

64. Composition according to any one of the preceding claims, **characterized in that** it comprises, in addition, pigments or nanopigments of metal oxides, coated or otherwise.

65. Composition according to Claim 64, **characterized in that** the said pigments or nanopigments are chosen from titanium, zinc, iron, zirconium or cerium oxides and mixtures thereof, coated or otherwise.

66. Composition according to any one of Claims 61 to 65, **characterized in that** it comprises, in addition, at least one agent for tanning and/or for artificial tanning of the skin.

67. Composition according to any one of Claims 61 to 66, **characterized in that** it comprises, in addition, at least one adjuvant chosen from fatty substances, organic solvents, thickeners, demulcents, opacifiers, stabilizers, emollients, antifoaming agents, moisturizing agents, perfumes, preservatives, polymers, fillers, sequestrants, propellants, alkalinizing or acidifying agents.

68. Composition according to any one of Claims 61 to 67, **characterized in that** it is a composition for protecting the human epidermis or an anti-sun composition and **in that** it is provided in the form of a nonionic vesicular dispersion, an emulsion, in particular an oil-in-water type emulsion, a cream, a milk, a gel, a gel cream, a suspension, a dispersion, a, powder, a solid stick, a foam or a spray.

69. Composition according to any one of Claims 61 to 67, **characterized in that** it is a make-up composition for the eyelashes, the eyebrows or the skin and **in that** it is provided in solid or pasty, anhydrous or aqueous form, in the form of an emulsion, a suspension or a dispersion.

70. Composition according to any one of Claims 61 to 67, **characterized in that** it is a composition intended for protecting the hair against ultraviolet rays and **in that** it is provided in the form of a shampoo, a lotion, a gel, an emulsion, a nonionic vesicular dispersion.

71. Use of the emulsion defined in any one of Claims 1 to 60 for the manufacture of cosmetic compositions for protecting the skin and/or the hair against ultraviolet radiation, in particular solar radiation.

72. Use of an amphiphilic polymer whose structure contains at least one fatty chain and at least one hydrophilic portion as defined in any one of the preceding claims for the manufacture of a photoprotective cosmetic or dermatological emulsion containing at least one organic UV-screening agent insoluble in the emulsion, with the aim of increasing the water resistance of its screening power (stability to water).

## Patentansprüche

1. Kosmetische oder dermatologische Emulsion, die mindestens ein Lichtschutzsystem enthält, das befähigt ist, die UV-Strahlung zu filtern, und das mindestens ein in der Emulsion unlösliches organisches UV-Filter in mikronisierter Form enthält, dessen mittlere Größe der Partikel im Bereich von 0,01 bis 2 µm liegt, **dadurch gekennzeichnet, daß** sie ferner mindestens ein amphiphiles Polymer enthält, das in seiner Struktur mindestens eine Fettkette und mindestens einen hydrophilen Bereich aufweist, wobei das Polymer von den C₈₋₁₆-Alkylpolyglucosiden verschieden ist.

2. Emulsion nach Anspruch 1, wobei das amphiphile Polymer anionisch, nichtionisch, kationisch oder amphoter vorliegt.

3. Emulsion nach Anspruch 2, wobei die amphiphilen Polymere unter den Polymeren ausgewählt sind, die mindestens eine hydrophile Einheit und mindestens eine Allylethereinheit mit Fettkette aufweisen.

4. Emulsion nach Anspruch 3, wobei die anionischen amphiphilen Polymere unter den Polymeren ausgewählt sind, deren hydrophile Einheit aus mindestens einer Vinylcarbonsäure besteht und deren Allylethereinheit mit Fettkette dem Monomer der folgenden Formel (I) entspricht:
CH₂=C(R')CH₂ O Bₙ R (I),
worin bedeuten: R' H oder CH₃, B Ethylenoxy, n Null oder eine ganze Zahl von 1 bis 100, R eine Kohlenwasserstoffgruppe, die unter Alkyl, Arylalkyl, Aryl, Alkylaryl oder Cycloalkyl mit 8 bis 30 Kohlenstoffatomen, vorzugsweise 10 bis 24 Kohlenstoffatomen und insbesondere 12 bis 18 Kohlenstoffatomen ausgewählt ist.

5. Emulsion nach Anspruch 4, wobei die hydrophile Einheit von Acrylsäure, Methacrylsäure und deren Gemischen gebildet wird.

6. Emulsion nach Anspruch 4 oder 5, wobei R' H, n 10 und R Stearyl (C₁₈) bedeutet.

7. Emulsion nach einem der Ansprüche 3 bis 6, wobei die anionischen amphiphilen Polymere unter den Polymeren ausgewählt sind, die aus 20 bis 60 Gew.-% Acrylsäure und/oder Methacrylsäure, 5 bis 60 Gew.-% Alkyl(meth)acrylaten, wobei es sich um niedere Alkylgruppen handelt, 2 bis 50 Gew.-% Allylether mit Fettkette der Formel (I) und 0 bis 1 Gew.-% Vernetzungsmittel gebildet werden.

8. Emulsion nach Anspruch 7, wobei das anionische amphiphile Polymer unter den vernetzten Terpolymeren von, Methacrylsäure, Ethylacrylat und dem Polyethylenglykolether (10 EO) von Stearylalkohol (Steareth-10) ausgewählt ist.

9. Emulsion nach Anspruch 2, wobei das anionische amphiphile Polymer unter den anionischen Polymeren ausgewählt ist, die mindestens eine hydrophile Einheit vom Typ einer olefinisch ungesättigten Carbonsäure und mindestens eine hydrophobe Einheit ausschließlich vom Typ eines C₁₀₋₃₀-Alkylesters einer ungesättigten Carbonsäure aufweisen.

10. Emulsion nach Anspruch 9, wobei die hydrophile Einheit vom Typ einer olefinisch ungesättigten Carbonsäure einem Monomer der folgenden Formel (II) entspricht: 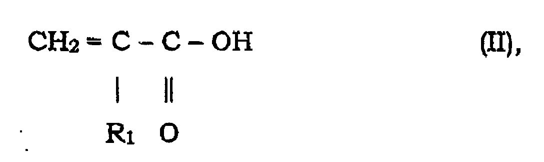 worin R₁ H oder CH₃ oder C₂H₅ bedeutet, d.h., es handelt sich um Acrylsäure-, Methacrylsäure- oder Ethacrylsäureeinheiten, und wobei die hydrophobe Einheit vom Typ eines C₁₀₋₃₀-Alkylesters einer ungesättigten Carbonsäure einem Monomer der folgenden Formel (III) entspricht: 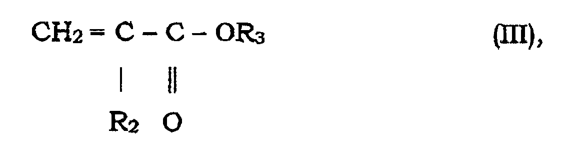 worin R₂ H oder CH₃ oder C₂H₅ und R₃ eine C₁₀₋₃₀-Alkylgruppe und vorzugsweise eine C₁₂₋₂₂-Alkylgruppe bedeutet.

11. Emulsion nach Anspruch 10, wobei die C₁₀₋₃₀-Alkylester von ungesättigten Carbonsäuren unter Laurylacrylat, Stearylacrylat, Decylacrylat und Isodecylacrylat und den entsprechenden Methacrylaten ausgewählt sind.

12. Emulsion nach einem der Ansprüche 9 bis 11, wobei das anionische Polymer ausgehend von einem Gemisch von Monomeren hergestellt wird, das enthält:
(i) im wesentlichen Acrylsäure,
(ii) einen Ester der folgenden Formel (III): 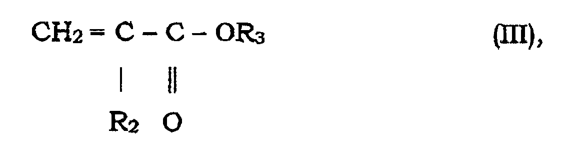 worin R₂ H oder CH₃ und R₃ eine Alkylgruppe mit 12 bis 22 Kohlenstoffatomen bedeutet, und
(iii) ein Vernetzungsmittel.

13. Emulsion nach einem der Ansprüche 9 bis 11, wobei das anionische amphiphile Polymer aus einem Monomerengemisch gebildet wird, das 95 bis 60 Gew.-% Acrylsäure, 4 bis 40 Gew.-% C₁₀₋₃₀-Alkylacrylat und 0 bis 6 Gew.-% polymerisierbares vernetzendes Monomer enthält.

14. Emulsion nach einem der Ansprüche 9 bis 11, wobei das anionische amphiphile Polymer aus einem Monomerengemisch gebildet wird, das 98 bis 96 Gew.-% Acrylsäure, 1 bis 4 Gew.-% C₁₀₋₃₀-Alkylacrylat und 0,1 bis 0,6 Gew.-% polymerisierbares vernetzendes Monomer enthält.

15. Emulsion nach Anspruch 2, wobei die nichtionischen amphiphilen Polymere ausgewählt sind unter:
(1) Cellulosen, die mit Gruppen modifiziert sind, die mindestens eine Fettkette aufweisen;
(2) Hydroxypropylguarverbindungen, die mit Gruppen modifiziert sind, die mindestens eine Fettkette aufweisen;
(3) Polyurethanpolyethern, die in ihrer Kette gleichzeitig hydrophile Sequenzen und hydrophobe Sequenzen aufweisen, wobei es sich nur um aliphatische Ketten und/oder cycloaliphatische Ketten und/oder aromatische Ketten handeln kann;
(4) Copolymeren von Vinylpyrrolidon und hydrophoben Monomeren mit Fettkette;
(5) Copolymeren von C₁₋₆-Alkylacrylaten oder C₁₋₆-Alkylmethacrylaten und amphiphilen Monomeren, die mindestens eine Fettkette aufweisen;
(6) Copolymeren von hydrophilen Methacrylaten und Acrylaten und hydrophoben Monomeren mit Fettkette.

16. Emulsion nach Anspruch 15, wobei die Cellulosen, die mit Gruppen modifiziert sind, die mindestens eine Fettkette aufweisen, ausgewählt sind unter:
- Hydroxyethylcellulosen, die mit Gruppen modifiziert sind, die mindestens eine Fettkette aufweisen, beispielsweise Alkyl-, Arylalkyl- oder Alkylarylgruppen oder deren Gemische, wobei die Alkylgruppen vorzugsweise 8 bis 22 Kohlenstoffatome enthalten;
- Hydroxyethylcellulosen, die mit Alkylphenolpolyalkylenglykolethergruppen modifiziert sind.

17. Emulsion nach Anspruch 15, wobei die Copolymere von Vinylpyrrolidon und hydrophoben Monomeren mit Fettkette ausgewählt sind unter:
- Vinylpyrrolidon/Hexadecen-Copolymeren;
- Vinylpyrrolidon/Eicosen-Copolymeren;

18. Emulsion nach Anspruch 15, wobei das Copolymer von C₁₋₆-Alkylacrylat oder C₁₋₆-Alkylmethacrylat und einem amphiphilen Monomer, das mindestens eine Fettkette aufweist, ein Copolymer von Methylacrylat und ethoxyliertem Stearylacrylat ist.

19. Emulsion nach Anspruch 15, wobei das Copolymer eines hydrophilen Acrylats oder Methacrylats und eines hydrophoben Monomers mit Fettkette ein Copolymer von Polyethylenglykolmethacrylat und Laurylmethacrylat ist.

20. Emulsion nach Anspruch 15, wobei die nichtionischen amphiphilen Polyurethanpolyether in ihrer Kette polyethoxylierte hydrophile Sequenzen enthalten.

21. Emulsion nach Anspruch 15, wobei die nichtionischen amphiphilen Polyurethanpolyether mindestens zwei hydrophobe Kohlenwasserstoffketten mit 6 bis 30 Kohlenstoffatomen enthalten, die durch eine hydrophile Sequenz voneinander getrennt sind, wobei die Kohlenwasserstoffketten Seitenketten oder Ketten am Ende der hydrophilen Sequenz sein können.

22. Emulsion nach einem der Ansprüche 20 bis 21, wobei die nichtionischen amphiphilen Polyurethanpolyether multisequenzielle Polymere und vorzugsweise Dreiblock-Polymere sind.

23. Emulsion nach einem der Ansprüche 20 bis 22, wobei die nichtionischen amphiphilen Polyurethanpolyether gepfropft oder sternförmig verzweigt sind.

24. Emulsion nach einem der Ansprüche 20 bis 23, wobei die nichtionischen amphiphilen Polyurethanpolyether unter den Dreiblock-Copolymeren ausgewählt sind, deren hydrophile Sequenz eine polyethoxylierte Kette mit 50 bis 1000 Ethylenoxidgruppen ist.

25. Emulsion nach Anspruch 2, wobei die kationischen amphiphilen Polymere unter den quaternisierten Cellulosederivaten und den Polyacrylaten mit aminierten Seitenketten ausgewählt sind.

26. Emulsion nach Anspruch 25, wobei die quaternisierten Cellulosederivate ausgewählt sind unter:
- quaternisierten Cellulosen, die mit Gruppen modifiziert sind, die mindestens eine Fettkette enthalten, vorzugsweise Alkyl-, Arylalkyl- oder Alkylarylgruppen mit mindestens 8 Kohlenstoffatomen und vorzugsweise 8 bis 30 Kohlenstoffatomen oder deren Gemische;
- quaternisierten Hydroxyethylcellulosen, die mit Gruppen modifiziert sind, die mindestens eine Fettkette enthalten, beispielsweise Alkyl-, Arylalkyl- oder Alkylarylgruppen mit mindestens 8 Kohlenstoffatomen und vorzugsweise 8 bis 30 Kohlenstoffatomen oder deren Gemische.

27. Emulsion nach Anspruch 25, wobei die Polyacrylate mit aminierten Seitenketten, die gegebenenfalls quaternisiert sind, hydrophobe Gruppen vom Typ Steareth 20 (polyethoxylierter (20) Stearylalkohol) aufweisen.

28. Emulsion nach Anspruch 2, wobei die amphiphilen amphoteren Polymere unter den vernetzten oder nicht vernetzten, verzweigten oder unverzweigten amphoteren Polymeren ausgewählt sind, die durch Copolymerisation der folgenden Verbindungen hergestellt werden können:
1) mindestens einem Monomer der Formel (IVa) oder (IVb): 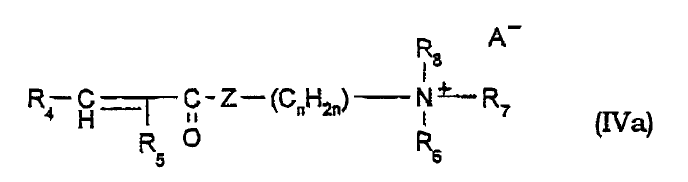 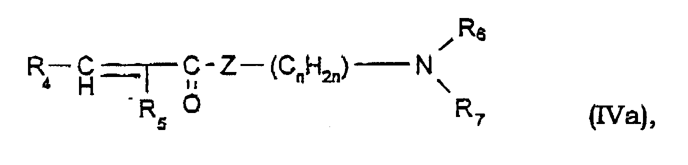 worin
die Gruppen R₄ und R₅, die identisch oder voneinander verschieden sind, Wasserstoff oder Methyl bedeuten,
die Gruppen R₆, R₇ und R₈, die identisch oder voneinander verschieden sind, eine geradkettige oder verzweigte Alkylgruppe mit 1 bis 30 Kohlenstoffatomen bedeuten,
Z die Gruppe NH oder ein Sauerstoffatom ist,
n eine ganze Zahl von 2 bis 5 bedeutet, und
A⁻ ein Anion ist, das von einer organischen oder anorganischen Säure abgeleitet ist, beispielsweise einMethosulfatanion oder ein Halogenidion, wie Chlorid oder Bromid;
2) mindestens einem Monomer der Formel (V): 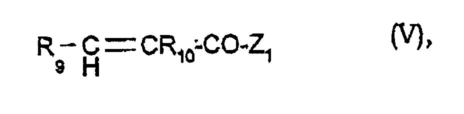 worin
die Gruppen R₉ und R₁₀, die identisch oder voneinander verschieden sind, Wasserstoff oder Methyl bedeuten,
Z₁ die OH-Gruppe oder die Gruppe NHC(CH₃)₂CH₂SO₃H bedeutet;
3) mindestens einem Monomer der Formel (VI): 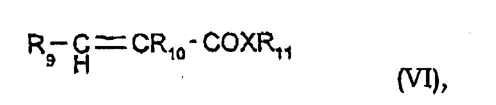 worin
die Gruppen R₉ und R₁₀, die identisch oder voneinander verschieden sind, Wasserstoff oder Methyl bedeuten,
X Sauerstoff oder Stickstoff bedeutet, und
die Gruppe R₁₁ eine geradkettige oder verzweigte Alkylgruppe mit 1 bis 30 Kohlenstoffatomen ist,
4) gegebenenfalls mindestens einem Vernetzungsmittel oder Mittel zur Verzweigung;
wobei mindestens eines der Monomere der Formeln (IVa), (IVb) oder (VI) mindestens eine Fettkette mit 8 bis 30 Kohlenstoffatomen aufweist und die Monomere der Formel (IVa), (IVb), (V) oder (VI) quaternisiert sein können, beispielsweise mit einem C₁₋₄-Alkylhalogenid oder einem C₁₋₄-Dialkylsulfat.

29. Emulsion nach einem der Ansprüche 1 bis 28, **dadurch gekennzeichnet, daß** es sich um eine einfache Öl-in-Wasser-Emulsion, eine einfache Wasser-in-Öl-Emulsion oder eine komplexe Emulsion handelt.

30. Emulsion nach Anspruch 29, **dadurch gekennzeichnet, daß** es sich um eine einfache Öl-in-Wasser-Emulsion handelt.

31. Emulsion nach Anspruch 30, wobei das amphiphile Polymer oder die amphiphilen Polymere die Aufgabe eines Emulgators übernehmen, um die Ölphase in der wässerigen Phase zu emulgieren.

32. Emulsion nach Anspruch 31, wobei das amphiphile Polymer oder die amphiphilen Polymere in einem Mengenanteil von 0,1 bis 20 % des Gesamtgewichts der Zusammensetzung und vorzugsweise in einem Mengenanteil von 0,5 bis 10 % vorliegen.

33. Emulsion nach einem der Ansprüche 1 bis 32, wobei das oder die unlöslichen, organischen UV-Filter unter den organischen UV-Filtern vom Oxanilidtyp, Triazintyp, Triazoltyp, Amidvinyltyp oder Cinnamidtyp ausgewählt sind.

34. Emulsion nach Anspruch 33, wobei die UV-Filter vom Oxanilidtyp der folgenden Struktur entsprechen: 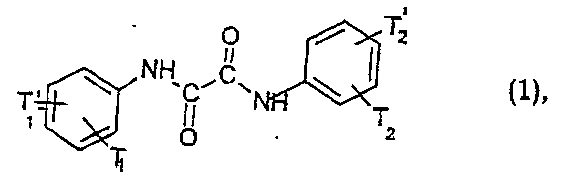 worin T₁, T'₁, T₂ und T'₂, die identisch oder voneinander verschieden sind, eine C₁₋₈-Alkylgruppe oder eine C₁₋₈-Alkoxygruppe bedeuten.

35. Emulsion nach Anspruch 33, wobei die UV-Filter vom Triazintyp unter den Verbindungen der folgenden allgemeinen Formel ausgewählt sind: 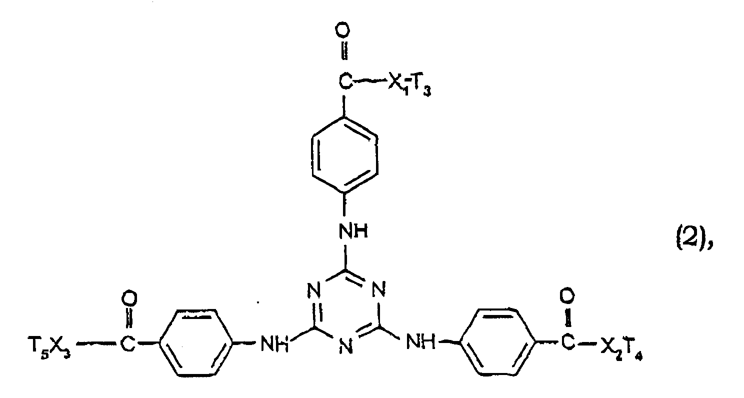 worin:
- die Gruppen X₁, X₂ und X₃, die identisch oder voneinander verschieden sind, Sauerstoff oder eine Gruppe -NZ bedeuten;
- die Gruppen Z, die identisch oder voneinander verschieden sind, Wasserstoff, eine geradkettige oder verzweigte C₁₋₁₈-Alkylgruppe oder eine C₅₋₁₂-Cycloalkylgruppe, die mit einer oder mehreren C₁₋₄-Alkylgruppen substituiert sein kann, bedeuten;
- die Gruppen T₃, T₄ und T₅, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer polyethoxylierten Gruppe mit 1 bis 6 Ethylenoxideinheiten, deren OH-Endgruppe methyliert ist; einer Gruppe der folgenden Formeln 3, 4 oder 5: 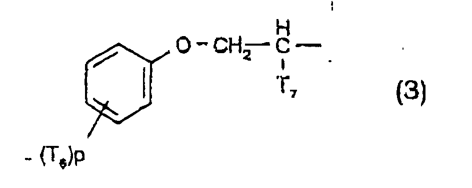 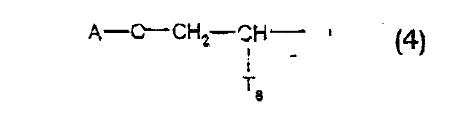 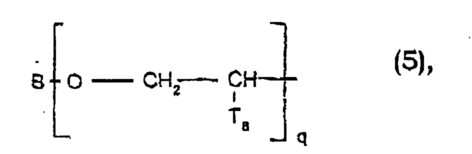 worin bedeuten:
- T₆ Wasserstoff oder Methyl;
- T₇ C₁₋₉-Alkyl;
- p 0 oder eine ganze Zahl von 1 bis 3;
- q eine ganze Zahl von 1 bis 10;
- A eine C₄₋₈-Alkylgruppe oder eine C₅₋₈-Cycloalkylgruppe;
- B ist ausgewählt unter: einer geradkettigen oder verzweigten C₁₋₈-Alkylgruppe; einer C₅₋₈-Cycloalkylgruppe; einer gegebenenfalls mit einer oder mehreren C₁₋₄-Alkylgruppen substituierten Arylgruppe;
- T₈ Wasserstoff oder Methyl.

36. Emulsion nach Anspruch 35, wobei das UV-Filter vom Triazintyp der folgenden Formel entspricht: 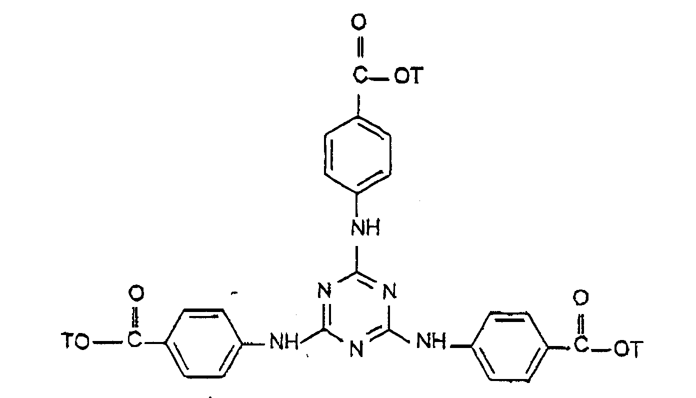 worin T eine 2-Ethylhexylgruppe bedeutet.

37. Emulsion nach Anspruch 35, wobei das UV-Filter vom Triazintyp der folgenden Formel entspricht: 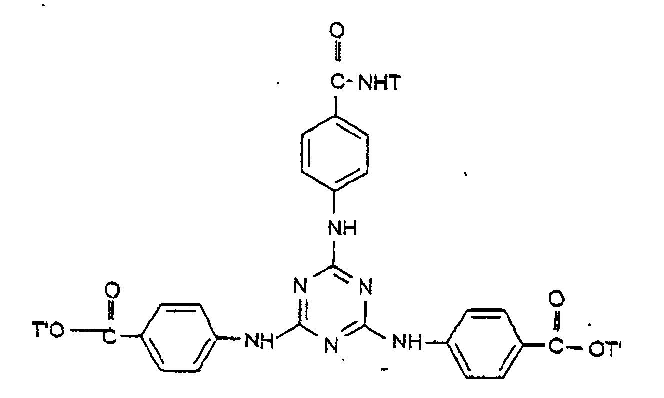 worin T' eine 2-Ethylhexylgruppe und T eine *t*-Butylgruppe bedeutet.

38. Emulsion nach Anspruch 33, wobei die UV-Filter vom Triazintyp unter den unlöslichen s-Triazinderivaten ausgewählt sind, die Benzalmalonat- und/oder Phenylcyanoacrylatgruppen enthalten.

39. Emulsion nach Anspruch 38, wobei die UV-Filter vom Triazintyp unter den folgenden Verbindungen ausgewählt sind:
- 2,4,6-Tris(diethyl-4'-aminobenzalmalonat)-s-triazin,
- 2,4,6-Tris(diisopropyl-4'-aminobenzalmalonat)-s-triazin,
- 2,4,6-Tris(dimethyl-4'-aminobenzalmalonat)-s-triazin,
- 2,4,6-Tris(ethyl-α-cyano-4-aminocinnamat)-s-triazin.

40. Emulsion nach Anspruch 33, wobei die UV-Filter vom Triazintyp unter den Verbindungen der folgenden Formel ausgewählt sind: 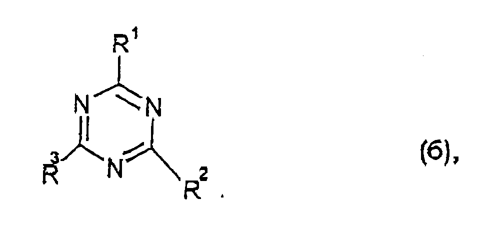 worin die Gruppen R¹, R² und R³ unabhängig voneinander Phenyl, Phenoxy oder Pyrrolo bedeuten, wobei die Phenyl-, Phenoxyoder Pyrrologruppen gegebenenfalls mit einem, zwei oder drei der folgenden Substituenten substituiert sind: OH, C₁₋₁₈-Alkyl oder C₁₋₁₈-Alkoxy, C₁₋₁₈-Carboxyalkyl, C₅₋₈-Cycloalkyl, Methylidencampher, -(CH=CH)ₙ(CO)-OR⁴, wobei R⁴ entweder C₁₋₁₈-Alkyl oder Cinnamyl bedeutet und n 0 oder 1 ist.

41. Emulsion nach Anspruch 33, wobei die UV-Filter vom Triazintyp unter den unlöslichen s-Triazinderivaten ausgewählt sind, die Benzotriazol- und/oder Benzothiazolgruppen enthalten.

42. Emulsion nach Anspruch 41, wobei die unlöslichen UV-Filter vom Triazintyp ausgewählt sind unter:
- 2,4,6-Tris[(3'-benzotriazol-2-yl-2'-hydroxy-5'-methyl)-phenylamino]-s-triazin,
- 2,4,6-Tris[(3'-benzotriazol-2-yl-2'-hydroxy-5'-*t*-octyl)-phenylamino]-s-triazin.

43. Emulsion nach Anspruch 33, wobei die unlöslichen organischen UV-Filter vom Triazoltyp der folgenden Formel 7 entsprechen: 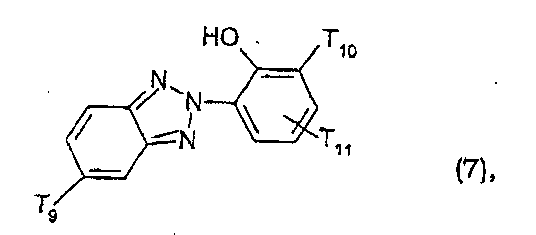 wobei T₉ ein Wasserstoffatom oder eine C₁₋₁₈-Alkylgruppe bedeutet und T₁₀ und T₁₁, die identisch oder voneinander verschieden sind, eine gegebenenfalls mit einer Phenylgruppe substituierte C₁₋₁₈-Alkylgruppe bedeuten.

44. Emulsion nach Anspruch 43, wobei die Verbindung der Formel 7 unter den folgenden Verbindungen ausgewählt ist:

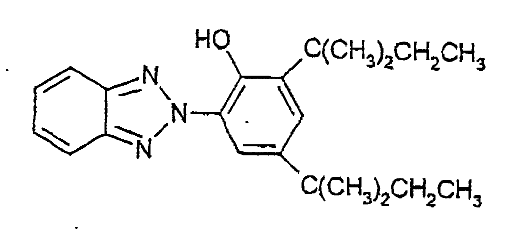

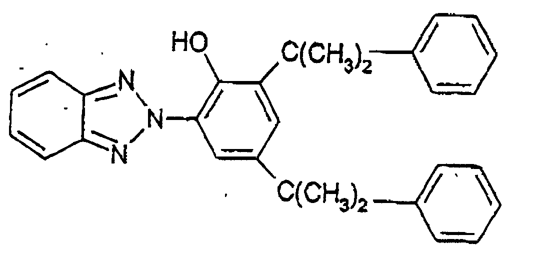

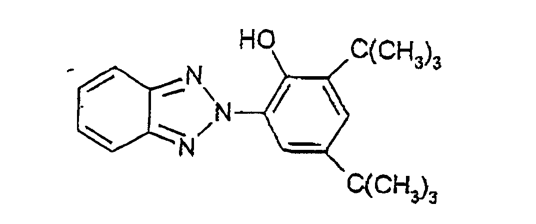

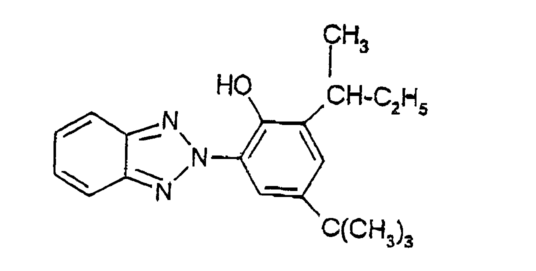

45. Emulsion nach Anspruch 33, wobei das unlösliche UV-Filter in mikronisierter Form das [2,4'-Dihydroxy-3-(2H-benzotriazol-2-yl)-5-(1,1,3,3-tetramethylbutyl)-2'-n-octoxy-5'-benzoyl]diphenylmethan der folgenden Struktur ist:

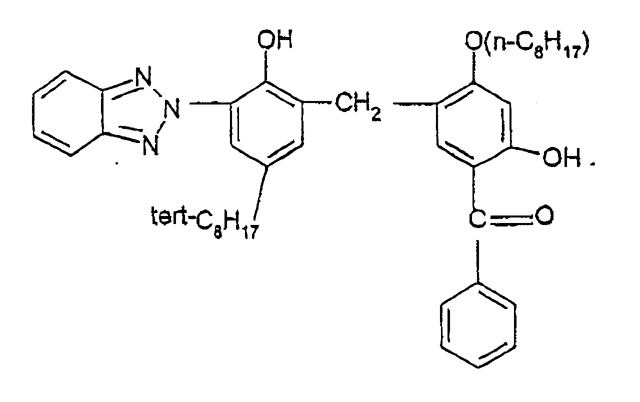

46. Emulsion nach Anspruch 33, wobei die organischen UV-Filter vom Triazoltyp unter den Methylen-bis(hydroxyphenylbenzotriazol)-Derivaten der folgenden Struktur ausgewählt sind: 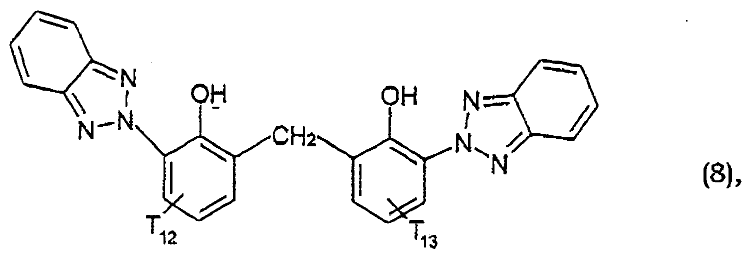 worin die Gruppen T₁₂ und T₁₃, die identisch oder voneinander verschieden sind, eine C₁₋₁₈-Alkylgruppe, die mit einer oder mehreren Gruppen substituiert sein kann, die unter den C₁₋₄-Alkylgruppen oder C₅₋₁₂-Cycloalkylgruppen ausgewählt sind, oder eine Arylgruppe bedeuten.

47. Emulsion nach Anspruch 46, wobei die Verbindung der Formel 8 unter den Verbindungen der folgenden Struktur ausgewählt ist:

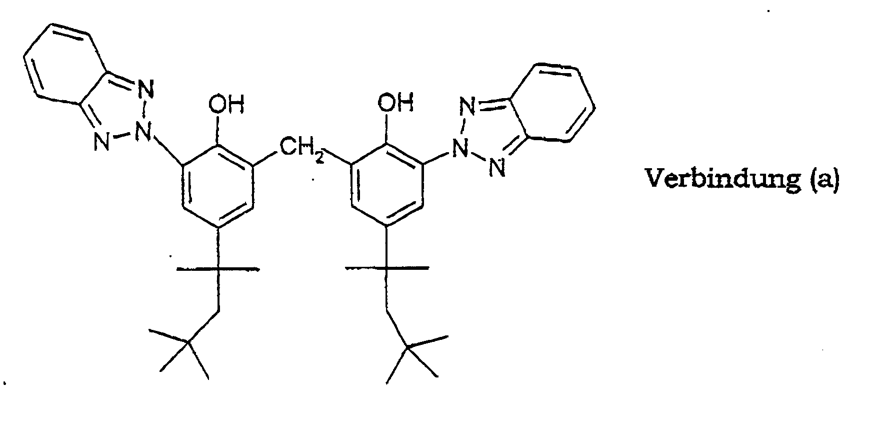

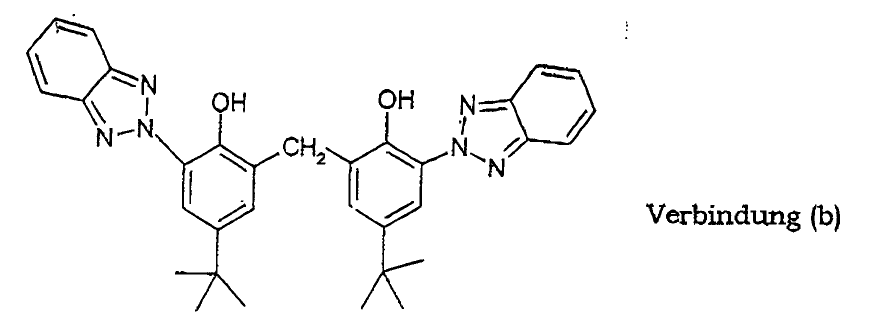

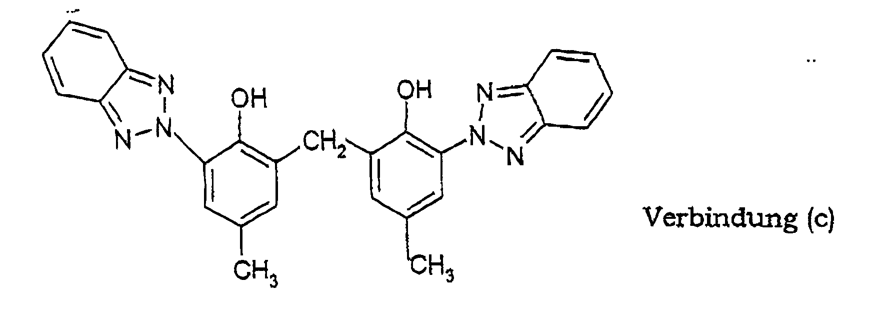

48. Emulsion nach Anspruch 33, wobei die unlöslichen organischen Filter vom Amidvinyltyp der folgenden Formel entsprechen
T₁₄-(Y)ᵣ-C(=O)-C(T₁₅)=C(T₁₆)-N(T₁₇)(T₁₈) (9),
worin T₁₄ eine C₁₋₁₈-Alkylgruppe und vorzugsweise eine C₁₋₅-Alkylgruppe, eine Phenylgruppe, die gegebenenfalls mit einer, zwei oder drei Gruppen substituiert ist, die ausgewählt sind unter: OH, C₁₋₁₈-Alkyl oder C₁₋₈-Alkoxy, oder -C(=O)-OT₁₉ bedeutet, wobei T₁₉ eine C₁₋₁₈-Alkylgruppe ist; die Gruppen T₁₅, T₁₆, T₁₇ und T₁₈, die identisch oder voneinander verschieden sind, eine C₁₋₁₈-Alkylgruppe und vorzugsweise eine C₁₋₅-Alkylgruppe oder ein Wasserstoffatom bedeuten; Y N oder O ist und r 0 oder 1 bedeutet.

49. Emulsion nach Anspruch 48, wobei die Verbindungen der Formel 9 ausgewählt sind unter:
- 4-Octylamino-3-penten-2-on;
- Ethyl-3-octylamino-2-butenoat;
- 3-Octylamino-1-phenyl-2-buten-1-on;
- 3-Dodecylamino-1-phenyl-2-buten-1-on.

50. Emulsion nach Anspruch 33, wobei die unlöslichen organischen Filter vom Cinnamamidtyp der folgenden Formel entsprechen: 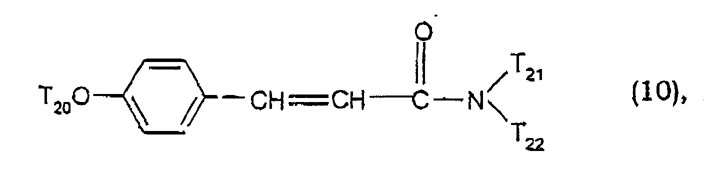 worin OT₂₀ eine Hydroxygruppe oder eine C₁₋₄-Alkoxygruppe ist, vorzugsweise Methoxy oder Ethoxy; T₂₁ Wasserstoff, C₁₋₄-Alkyl und vorzugsweise Methyl oder Ethyl bedeutet; T₂₂ eine Gruppe -(CONH)ₛ-phenyl, wobei s 0 oder 1 bedeutet und die Phenylgruppe mit einer, zwei oder drei Gruppen substituiert sein kann, die unter OH, C₁₋₁₈-Alkyl oder C₁₋₈-Alkoxy ausgewählt sind oder -C(=O)-OT₂₃ bedeutet, wobei T₂₃ eine C₁₋₁₈-Alkylgruppe und noch bevorzugter eine Phenylgruppe, 4-Metboxyphenyl oder Phenylaminocarbonyl ist.

51. Emulsion nach Anspruch 33, wobei das unlösliche UV-Filter ein Cinnamamid-Dimer ist.

52. Emulsion nach Anspruch 51, wobei das unlösliche UV-Filter die Verbindung der folgenden Struktur ist:

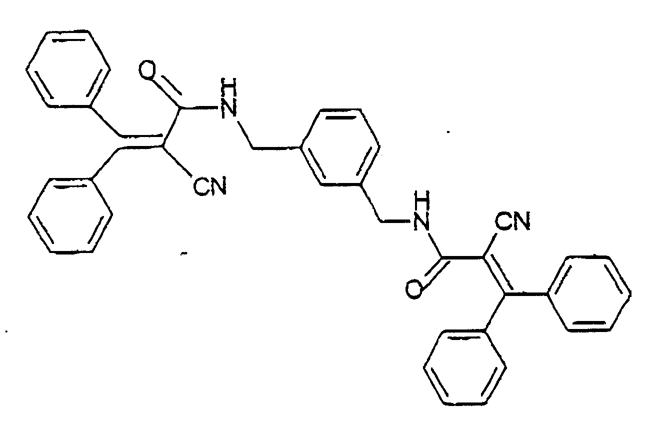

53. Emulsion nach einem der Ansprüche 1 bis 32, wobei die unlöslichen UV-Filter mehrwertige Metallsalze von organischen Sulfonfiltern oder Carboxyfiltern sind.

54. Emulsion nach Anspruch 33, wobei die unlöslichen UV-Filter unter den mehrwertigen Metallsalzen von sulfonierten Benzylidencampherderivaten; mehrwertigen Metallsalzen von sulfonierten Benzimidazolderivaten; und mehrwertigen Metallsalzen von Zimtsäurederivaten ausgewählt sind.

55. Emulsion nach einem der Ansprüche 1 bis 32, wobei es sich bei den unlöslichen UV-Filtern um Komplexe von mehrwertigen Metallen oder Ammonium oder substituiertem Ammonium und organischen UV-A- und/oder UV-B-Filtern handelt.

56. Emulsion nach einem der Ansprüche 1 bis 55, wobei das oder die unlösliche(n) UV-Filter, die in unlöslicher, mikronisierter Form vorliegen, eine mittlere Größe der Partikel im Bereich von 0,02 bis 1,5 µm aufweisen.

57. Emulsion nach Anspruch 56, wobei das oder die unlösliche(n) UV-Filter, die in unlöslicher, mikronisierter Form vorliegen, eine mittlere Größe der Partikel im Bereich von 0,03 bis 1,0 µm aufweisen.

58. Emulsion nach einem der Ansprüche 1 bis 57, **dadurch gekennzeichnet, daß** das oder die unlösliche(n) UV-Filter in mikronisierter Form durch Zerkleinern eines unlöslichen organischen Filters, das in Form von groben Partikel vorliegt, in Gegenwart eines grenzflächenaktiven Stoffes hergestellt werden kann.

59. Emulsion nach Anspruch 58, wobei der grenzflächenaktive Stoff unter den Alkylpolyglucosiden der Struktur CₙH₂ₙ₊₁O(C₆H₁₀O₅)ₓH ausgewählt ist, worin n eine ganze Zahl von 8 bis 16 bedeutet und x der mittlere Polymerisationsgrad der Einheit (C₆H₁₀O₅) ist, und im Bereich von 1,4 bis 1,6 liegt.

60. Emulsion nach einem der Ansprüche 58 bis 59, **dadurch gekennzeichnet, daß** der grenzflächenaktive Stoff in einer Konzentration von 1 bis 50 Gew.-%, bezogen auf das in mikronisierter Form vorliegende, unlösliche Filter verwendet wird.

61. Kosmetische oder dermatologische Zusammensetzung, die zum Lichtschutz der Haut und/oder der Haare vorgesehen ist, **dadurch gekennzeichnet, daß** sie in einem kosmetisch akzeptablen Träger eine Emulsion nach einem der Ansprüche 1 bis 60 enthält.

62. Zusammensetzung nach Anspruch 61, **dadurch gekennzeichnet, daß** sie ferner ein oder mehrere zusätzliche, im UV-A- und/oder UV-B-Bereich wirksame organische Filter enthält, die in mindestens einer der Phasen der Zusammensetzung löslich sind.

63. Zusammensetzung nach Anspruch 62, **dadurch gekennzeichnet, daß** die zusätzlichen organischen Filter ausgewählt sind unter: Zimtsäurederivaten, Salicylsäurederivaten, Campherderivaten, Triazinderivaten, Dibenzoylmethanderivaten, Benzophenonderivaten, β,β'-Diphenylacrylatderivaten, von α-Alkylstyrol abgeleiteten Dimeren, Benzimidazolderivaten, Bisbenzoazolylderivaten, p-Aminobenzoesäurederivaten, polymeren Filtern und Siliconfiltern.

64. Zusammensetzung nach einem der vorhergehenden Ansprüche, **dadurch gekennzeichnet, daß** sie außerdem Pigmente oder Nanopigmente von Metalloxiden enthält, die gegebenenfalls umhüllt sind.

65. Zusammensetzung nach Anspruch 64, **dadurch gekennzeichnet, daß** die Pigmente oder Nanopigmente unter den Oxiden von Titan, Zink, Eisen, Zirconium, Cer und deren Gemischen ausgewählt sind, wobei die Pigmente oder Nanopigmente gegebenenfalls umhüllt sind.

66. Zusammensetzung nach einem der Ansprüche 61 bis 66, **dadurch gekennzeichnet, daß** sie außerdem mindestens ein Mittel zur Bräunung und/oder künstlichen Braunfärbung der Haut enthält.

67. Zusammensetzung nach einem der Ansprüche 61 bis 66, **dadurch gekennzeichnet, daß** sie ferner mindestens einen Zusatzstoff enthält, der unter den Fettsubstanzen, organischen Lösungsmitteln, Verdickungsmitteln, Mitteln für die Geschmeidigkeit, Trübungsmitteln, Stabilisierungsmitteln, Emollientien, Antischaummitteln, Hydratisierungsmitteln, Parfums, Konservierungsmitteln, Polymeren, Füllstoffen, Maskierungsmitteln, Treibmitteln, Alkalisierungsmitteln oder Ansäuerungsmitteln ausgewählt ist.

68. Zusammensetzung nach einem der Ansprüche 61 bis 67, **dadurch gekennzeichnet, daß** es sich um eine Zusammensetzung zum Schutz der menschlichen Epidermis oder ein Sonnenschutzmittel handelt und dadurch, daß sie als nichtionische Vesikeldispersion, Emulsion und insbesondere Emulsion vom Ölin-Wasser-Typ, Creme, Milch, Gel, Gelcreme, Suspension, Dispersion, Pulver, fester Stift, Schaum oder Spray vorliegt.

69. Zusammensetzung nach einem der Ansprüche 61 bis 67, **dadurch gekennzeichnet, daß** es sich um eine Zusammensetzung zum Schminken der Wimpern, Augenbrauen oder der Haut handelt und dadurch, daß sie in fester oder pastöser, wasserfreier oder wässeriger Form, als Emulsion, Suspension oder Dispersion vorliegt.

70. Zusammensetzung nach einem der Ansprüche 61 bis 67, **dadurch gekennzeichnet, daß** es sich um eine Zusammensetzung handelt, die zum Schutz der Haare gegen UV-Strahlung vorgesehen ist, und dadurch, daß sie als Haarwaschmittel, Lotion, Gel, Emulsion oder nichtionische Vesikeldispersion vorliegt.

71. Verwendung einer Emulsion nach einem der Ansprüche 1 bis 60 zur Herstellung von kosmetischen Zusammensetzungen zum Schutz der Haut und/oder der Haare gegen UV-Strahlung und insbesondere gegen Sonnenlicht.

72. Verwendung eines amphiphilen Polymers, das in seiner Struktur mindestens eine Fettkette und mindestens einen hydrophilen Bereich aufweist, nach einem der vorhergehenden Ansprüche zur Herstellung einer kosmetischen oder dermatologischen Emulsion zum Lichtschutz, die mindestens ein in der Emulsion unlösliches, organisches UV-Filter enthält, um im Hinblick auf das Filtervermögen die Wasserbeständigkeit zu erhöhen (Remanenz gegenüber Wasser).
